# EUROPEAN PATENT APPLICATION

(11) **EP 4 760 260 A2**
(43) Date of publication of application: **17.06.2026**
(21) Application number: 25226080.7
(22) Date of filing: 15.03.2022
(51) Int. Cl.: G01N 33/68

(54) **SYSTEM AND METHOD FOR ONLINE DETECTION OF A POST-TRANSLATIONAL MODIFICATION OF A POLYPEPTIDE**

(30) Priority: 16.03.2021 US 202163200576 P
(62) Divisional of application: 22715706.2
(71) Applicant: Astrazeneca AB, 151 85 Södertälje (SE)
(72) Inventor: AHN, Joomi, Gaithersburg, MD 20878 (US); ALBARGHOUTHI, Methal, Gaithersburg, MD 20878 (US)
(74) Representative: AstraZeneca Intellectual Property

(57) **Abstract**

Provided herein is a method and system for monitoring a post-translational modification and glycosylation of a polypeptide, and in particular, to an online i.e. automated method for real-time monitoring of a post-translational modification of a recombinant protein produced in cell culture. The methods and systems to detect and quantify post-translational modifications of proteins involve flow injection analysis (FIA), affinity columns, spectrometry, digestion and peptide mapping.

## Description

### CROSS-REFERENCE TO RELATED APPLICATIONS

This application claims benefit under 35 U.S.C. §119(e) of U.S. Provisional Patent Application No. 63/200,576, filed March 16, 2021, which is incorporated by reference herein in its entirety for all purposes.

### FIELD OF THE INVENTION

The present disclosure relates to a method for detecting a post-translational modification of a polypeptide, and in particular, to an online method for real-time detection of a post-translational modification of a recombinant protein produced in cell culture.

### BACKGROUND OF THE INVENTION

Biopharmaceuticals, which include therapeutic proteins, such as fusion proteins, growth factors, cytokines, enzymes, hormones, and monoclonal antibodies (mAbs) represent the fastest growing sector of the pharmaceutical industry. Typically, biopharmaceuticals are recombinantly produced in cell culture using genetically engineered host cells, including microbial systems such as *Escherichia coli* or *Saccharomyces cerevisiae,* fungal systems such as *Aspergillus,* and mammalian systems such as Chinese Hamster Ovary (CHO), NS0 or Sp2/0 host cells. Mammalian systems are often preferred because many biopharmaceuticals are too large and complex to be made by microorganisms or because the biopharmaceutical requires a post-translational modification (PTM) that is only possible in a mammalian host cell. However, PTMs can affect physicochemical properties of the biomolecule, which can impact activity, protein-protein interactions, biological function, efficacy and stability and can result in varying levels of protein heterogeneity. Consequently, PTMs are Critical Quality Attributes (CQAs) that are characterized, controlled, and monitored during process development and manufacturing of biopharmaceuticals.

Common PTMs include glycosylation, deamidation, isomerization, and oxidation, incomplete disulfide bond formation, glycation, N-terminal glutamine cyclization and C-terminal lysine processing. Of these modifications, the most common and naturally occurring are phosphorylation, acetylation, glycosylation (O- and N-linked), deamidation, oxidation, hydroxylation, methylation, ubiquitination, pyroglutamic acid, sulfation as well as truncation. Virág et al. (2020) "Current Trends in the Analysis of Post-translational Modifications," Chromatographia. 83:1-10 (doi.org/10.1007/s10337-019-03796-9).

Glycosylation is a common PTM that involves the attachment of glycans at specific sites on a protein, for example, at Asparagine residues (*N*-linked glycosylation) or at Serine (Ser) or Threonine (Thr) residues (*O*-linked glycosylation). *N*-linked glycosylation occurs at a consensus sequence (Asn-X-Ser/Thr, where X is any amino acid except proline) and starts in the endoplasmic reticulum (ER), where a glycan chain (Glc₃Man₉GlcNAc₂) is added to the Asn residue of the consensus sequence. After the glycan chain is trimmed by various glycoside hydroxylase enzymes, the glycoprotein is transported to the Golgi apparatus where the glycans are subjected to further trimming and modifications, generating a variety of *N*-glycan structures. *O*-linked glycans are usually attached to serine (Ser) or threonine (Thr) residues via an α-*O-*glycosidic bond formed between *N*-acetylgalactosamine (GalNAc) and the hydroxyl group (-OH) of the Ser or Thr residue. *O*-glycan biosynthesis is initiated in the Golgi by the transfer of GalNAc from UDP-GalNAc to the Ser or Thr residue. The *O*-GalNAc precursor is processed to produce a variety of *O*-GalNAc glycans. Zhang et al. (2016) "Glycan analysis of therapeutic glycoproteins," MAbs. 8(2):205-215.

Conventional processes for analyzing PTMs involve the manual collection of a sample at the end of the cell culture and delivery of the sample to a testing laboratory, where it is concentrated, purified and prepared for analysis. The turnaround time for conventional processes can be from 3 to 5 days, which can result in delays and increased costs in development and manufacturing. Because the analysis is typically performed at the end of the process, the results reflect an aggregate result of the process. Additionally, the entire process needs to be repeated, starting with the cell culture, if the product does not meet the required specifications. Therefore, there is a desire for analytic methods that can be performed in real-time, to characterize a product during the course of manufacturing, such that manufacturing parameters can be adjusted to keep the product within the desired specifications.

Tharmalingham et al. (2015) "A Framework for Real-Time Glycosylation Monitoring (RT-GM) in Mammalian Cell Culture," Biotech. Bioeng. 112(6):1146-1154, describe a method for monitoring glycosylation in real-time using a micro sequential injection system as a sample preparation platform that is coupled with an ultra-performance liquid chromatography (UPLC) system for real-time monitoring of an antibody glycan profile. For glycan analysis, glycans were released from the antibody enzymatically using PNGase F (P0704, New England BioLabs, Ipswich, MA) and separated using a UPLC column.

There remains a need for improved processes for real-time monitoring of post-translational modifications (PTMs) of recombinantly produced proteins.

### SUMMARY OF THE INVENTION

The present invention relates to methods and systems for detecting, identifying or monitoring a post-translational modification (PTM) of a recombinantly produced protein, and in particular, to an online method or system for real-time monitoring of PTM of a recombinantly produced protein during cell culture.

In one aspect, a method is provided for detecting a post-translational modification of a protein, wherein the method is carried out by a flow injection analyzer (FIA) controlled by at least one processor. In one aspect, the FIA is a sequential injection analyzer (SIA). In one aspect, the FIA is a direct injection analyzer (DIA). In one aspect, the method includes:
(a) receiving a first sample that includes the protein in a holding reservoir;
(b) delivering, via the FIA, a first volume of a binding buffer to an affinity column in the FIA;
(c) transferring, via the FIA, the first sample from the holding reservoir to the affinity column;
(d) delivering, via the FIA, a first volume of an elution buffer to the affinity column and thereby eluting the protein in the first sample from the affinity column to form a first eluate;
(e) transferring, via the FIA, the first eluate to a protein concentration detection unit;
(f) determining, by the at least one processor, a concentration of the protein in the first eluate;
(g) receiving a second sample that includes the protein in the holding reservoir;
(h) delivering, via the FIA, a second volume of the binding buffer to the affinity column;
(i) transferring, via the FIA, the second sample from the holding reservoir to the affinity column;
(j) delivering, via the FIA, a second volume of an elution buffer to the affinity column thereby eluting the protein in the second sample from the affinity column to form a second eluate;
(k) transferring, via the FIA, the second eluate to a digestion chamber;
(l) delivering, via the FIA, a digestion reagent to digest the protein in the second eluate to form a peptide mixture; and
(m) transferring, via the FIA, the peptide mixture to a peptide mapping unit and detecting the post-translational modification of the protein.

In one aspect, the first and second samples are obtained from a cell culture. In one aspect, the cell culture is in a bioreactor. In one aspect, the bioreactor is a batch, fed-batch or perfusion bioreactor.

In one aspect, the affinity column includes a protein A column.

In one aspect, determining the concentration of the protein in the first eluate in (f) is performed by the at least one processor according to a predetermined calibration curve.

In one aspect, the elution time of the protein in the first sample from the affinity column to form the first eluate is controlled by the at least one processor based on a predetermined elution time. In one aspect, the elution time of the protein in the second sample from the affinity column to form the second eluate is controlled by the at least one processor based on a predetermined elution time. In one aspect, the predetermined elution times for the protein in the first and second samples are the same. In one aspect, the predetermined elution times for the protein in the first and second samples is determined experimentally. In one aspect, the predetermined elution times for the protein in the first and second samples is determined experimentally prior to performing the online method described herein.

In one aspect, the protein concentration detection unit includes a spectrophotometer. In one aspect, the spectrophotometer measures UV absorbance of the first eluate and, by the at least one processor, calculates a protein concentration based on a predetermined calibration curve. In one aspect, the calibration curve is determined experimentally. In one aspect, the calibration curve is determined experimentally prior to performing the online method described herein. In one aspect, the spectrophotometer measures UV absorbance of the first eluate at 280 nm and, by the at least one processor, calculates a protein concentration based on a calibration curve. In one aspect, the spectrophotometer measures UV absorbance of the first eluate at 205 nm and, by the at least one processor, calculates a protein concentration based on a calibration curve.

In one aspect, the protein in the second eluate is enzymatically digested in (l). In one aspect, enzymatic digestion of the protein includes contacting, via the FIA, the second eluate with a proteolytic enzyme selected from: trypsin, chymotrypsin, pepsin, thermolysin, papain, pronase, endopeptidase Arg-C, peptidyl-Asp metallo endopeptidase (endopeptidase Asp-N), Glutamyl endopeptidase (Glu-C endopeptidase), and Lysyl endopeptidase (Lys-C endopeptidase). In one aspect, the proteolytic enzyme includes trypsin.

In one aspect, the proteolytic enzyme includes heat-stable trypsin. In one aspect, the method includes:
(i) introducing, via the FIA, a digestion reagent to the second eluate to form a digestion mixture;
(ii) mixing the digestion mixture; and
(iii) incubating the digestion mixture.

In one aspect, the digestion reagent includes a digestion buffer, a reducing agent, and a proteolytic enzyme. In one aspect, the digestion buffer has a pH from about 6.0 to about 7.5. In one aspect, the reducing agent includes Tris(2-carboxyethyl)phosphine (TCEP). In one aspect, the second eluate is contacted with an amount of proteolytic enzyme determined by the at least one processor based on the concentration of the first eluate. In one aspect, the proteolytic enzyme includes trypsin. In one aspect, the proteolytic enzyme includes heat-stable trypsin. In one aspect, the second eluate is contacted with a proteolytic enzyme at a ratio of enzyme:protein from about 1:10 to about 1:100. In one aspect, the second eluate is contacted with a proteolytic enzyme at a ratio of enzyme:protein from about 1:20 to about 1:30. In one aspect, the second eluate is contacted with trypsin at a ratio of trypsin:protein from about 1:10 to about 1:100. In one aspect, the second eluate is contacted with trypsin at a ratio of trypsin:protein from about 1:20 to about 1:30.

In one aspect, incubating the digestion mixture includes incubating at a temperature from about 36°C to about 85°C for about 5 minutes to about 30 minutes. In one aspect, incubating the digestion mixture includes incubating at a temperature from about 50°C to about 75°C for about 15 minutes to about 30 minutes. In one aspect, incubating the digestion mixture includes incubating at a temperature from about 70°C to about 72°C for about 15 minutes to about 30 minutes.

In one aspect, the proteolytic enzyme includes heat-stable trypsin, and the method includes:
(i) introducing, via the FIA, a first amount of a digestion reagent that includes digestion buffer, reducing agent and heat-stable trypsin to the second eluate and mixing to form a first digestion mixture;
(ii) incubating the first digestion mixture at a temperature of about 70°C to about 75°C for about 10 minutes to about 20 minutes to form a first incubated mixture;
(iii) introducing, via the FIA, a second amount of heat-stable trypsin to the incubated mixture and mixing to form a second digestion mixture; and
(iv) incubating the second digestion mixture at a temperature of about 70°C to about 75°C for about 10 minutes to about 20 minutes to form a peptide mixture.

In one aspect, a second amount of digestion buffer is added to the first incubated mixture, via the FIA, with the second amount of heat-stable trypsin in (iii).

In one aspect, the peptide mapping unit includes a mass spectrometer (MS). In one aspect, detecting post-translational modifications includes a peptide mapping analysis performed using mass spectrometry (MS). In one aspect, detecting a post-translation modification includes a peptide mapping analysis performed using liquid chromatography/mass spectrometry (LC/MS). In one aspect, liquid chromatography includes ultra-performance liquid chromatography (UPLC).

In one aspect, the method is performed online.

In one aspect, the post-translational modification includes glycosylation, oxidation, deamidation, isomerization, glycation, N-terminus leader sequence, N-terminal cyclization, C-terminus lysine retention, amide formation or a combination thereof. In one aspect, glycosylation includes N-linked glycosylation, O-linked glycosylation or a combination thereof.

In one aspect, a method of producing a recombinant protein is provided. In one aspect, the method includes:
(a) culturing a host cell under conditions in which the recombinant protein is expressed;
(b) detecting a post-translational modification of the protein according to a method described herein;
(c) modifying one or more cell culture parameters based on the post-translational modification detected in (b).

In one aspect, the host cell is cultured in a bioreactor that is a batch, fed-batch or perfusion bioreactor. In one aspect, the host cell is a mammalian host cell. In one aspect, the host cell is a CHO, HEK 293, COS, NS0, SP2 or PER.C6 host cell.

In one aspect, one of more of the following cell culture parameters are modified based on the post-translational modifications detected using a method described herein: pH; CO₂ level; amount of dissolved oxygen (dO₂); temperature; amount or type of nutrient; presence and types of glycan precursors, or a combination thereof.

In one aspect, the protein includes at least 1000 amino acid residues. In one aspect, the protein is recombinantly produced. In one aspect, the protein includes a glycoprotein. In one aspect, the protein includes a therapeutic protein. In one aspect, the protein includes a therapeutic antibody or antigen binding fragment thereof. In one aspect, the protein includes a fusion protein.

In one aspect, a recombinant protein is provided that is produced by a method described herein. In one aspect, a pharmaceutical composition is provided that includes a recombinant protein produced by a method described herein and a pharmaceutically acceptable carrier.

In one aspect, an online method for monitoring post-translational modifications of a protein is provided. In one aspect, the method is carried out by a flow injection analyzer (FIA). In one aspect, the FIA is a sequential injection analyzer (SIA). In one aspect, the FIA is a direct injection analyzer (DIA). In one aspect, the FIA includes a bi-directional pump controlled by at least one processor. In one aspect, the FIA includes a syringe pump and a multi-port valve controlled by at least one processor. In one aspect, the FIA includes more than one syringe pump and more than one multi-port valve. In one aspect, the FIA includes two syringe pumps and two multi-port valves.

In one aspect, the method includes:
(a) receiving a first sample that includes the protein in a holding reservoir of the FIA;
(b) introducing, by action of the multi-port valve, a first volume of a binding buffer into an affinity column in the FIA;
(c) advancing, by action of a syringe pump, the first sample from the holding reservoir to the affinity column and allowing the protein in the first sample to bind to the affinity column;
(d) introducing, by action of the multi-port valve, a first volume of an elution buffer into the affinity column to elute the bound protein to form a first eluate;
(e) advancing, by action of the syringe pump, the first eluate to a spectrophotometer in the FIA;
(f) determining, by the at least one processor and according to a measured UV absorbance, a concentration of protein in the first eluate;
(g) receiving a second sample that includes the protein in the holding reservoir;
(h) introducing, by action of the multi-port valve, a second volume of the binding buffer into the affinity column;
(i) advancing, by action of the syringe pump, the second sample from the holding reservoir to the affinity column and allowing the protein in the second sample to bind to the affinity column;
(j) introducing, by action of the multi-port valve, a second volume of elution buffer into the affinity column to elute the bound protein to form a second eluate;
(k) advancing, by action of the syringe pump, the second eluate to a digestion chamber in the FIA; and
(l) introducing, by action of the multi-port valve, a digestion reagent into the second eluate in the digestion chamber and digesting the protein to form a peptide mixture; and
(m) advancing, by action of the syringe pump, the peptide mixture to a peptide mapping unit and detecting a post-translational modification of the protein.

In one aspect, wherein digesting the protein includes:
(i) introducing, by action of the multi-port valve, a digestion buffer, a reducing agent, and a proteolytic enzyme into the second eluate in the digestion chamber and mixing to form a first digestion mixture;
(ii) incubating the first digestion mixture in the digestion chamber to form a first incubated mixture;
(iii) introducing, by action of the multi-port valve, a second volume of digestion buffer and proteolytic enzyme into the incubated mixture to form a second digestion mixture; and
(iv) incubating the second digestion mixture in the digestion chamber to form a peptide mixture.

In one aspect, determining the concentration of the protein in the first eluate in (f) is performed by the at least one processor according to a predetermined calibration curve. In one aspect, the protein in the first sample is eluted from the affinity column to form a first eluate based on a predetermined elution time. In one aspect, the protein in the second sample is eluted from the affinity column to form a second eluate based on a predetermined elution time.

In one aspect, the digestion buffer has a pH from about 6.0 to about 7.5. In one aspect, the reducing agent is Tris(2-carboxyethyl)phosphine (TCEP). In one aspect, an amount of proteolytic enzyme introduced into the digestion chamber is determined by the at least one processor based on the concentration of the first eluate (previously determined). In one aspect, the proteolytic enzyme includes trypsin. In one aspect, trypsin includes heat-stable trypsin. In one aspect, the second eluate is contacted with trypsin at a ratio of trypsin:protein from about 1:10 to about 1:100. In one aspect, the second eluate is contacted with trypsin at a ratio of trypsin:protein from about 1:20 to about 1:30.

In one aspect, the first digestion mixture is incubated at a temperature from about 36°C to about 85°C for about 5 minutes to about 30 minutes. In one aspect, the first digestion mixture is incubated at a temperature from about 50°C to about 75°C for about 15 minutes to about 30 minutes. In one aspect, the first digestion mixture is incubated at a temperature from about 70°C to about 72°C for about 15 minutes to about 30 minutes.

In one aspect, the second digestion mixture is incubated at a temperature from about 36°C to about 85°C for about 5 minutes to about 30 minutes. In one aspect, the second digestion mixture is incubated at a temperature from about 50°C to about 75°C for about 15 minutes to about 30 minutes. In one aspect, the second digestion mixture is incubated at a temperature from about 70°C to about 72°C for about 15 minutes to about 30 minutes.

In one aspect, the proteolytic enzyme includes heat-stable trypsin, and the method includes:
(i) introducing, by action of the multi-port valve, a first amount of digestion buffer, reducing agent and heat-stable trypsin into the second eluate and mixing to form a first digestion mixture;
(ii) incubating the first digestion mixture at a temperature of about 70°C to about 75°C for about 10 minutes to about 20 minutes to form an incubated mixture;
(iii) introducing, by action of the multi-port valve, a second amount of digestion buffer and heat-stable trypsin to the incubated mixture and mixing to form a second digestion mixture; and
(iv) incubating the second digestion mixture at a temperature of about 70°C to about 75°C for about 10 minutes to about 20 minutes to form a peptide mixture.

In one aspect, the protein includes at least about 1000 amino acid residues.

In one aspect, the peptide mixture includes peptides that includes less than about 100 amino acids.

In one aspect, an online system for monitoring a post-translational modification of a protein of interest is provided. In one aspect, the system includes:
(a) a holding reservoir configured to receive a first and a second sample that each include the protein of interest;
(b) a first syringe pump configured to advance the first and second sample from the holding reservoir through the flow injection analyzer (FIA);
(c) a first multi-port valve in fluid communication with first syringe pump;
(d) an affinity column in fluid communication with the first multi-port valve and configured to receive the first and second sample from the holding reservoir;
(e) a spectrophotometer in the FIA configured to receive a first eluate from the affinity column, wherein the first eluate comprises protein from the first sample, wherein the spectrophotometer is configured to determine a UV absorbance, and calculate a protein concentration of the first sample based on a predetermined calibration curve;
(f) a second syringe pump configured to advance a second eluate from the affinity column to a digestion chamber, wherein the second eluate comprises protein from the second sample, and wherein the protein in the second eluate is digested based on the protein concentration of the first sample determined in (e); and
(g) a second injection multi-port valve in fluid communication with the second syringe pump.

### BRIEF DESCRIPTION OF THE FIGURES

FIG. 1 is a schematic diagram of a system configured to provide process implementation of automated methods consistent with the method described herein.
FIG. 2 is a schematic diagram of a computer system configured to provide process control of automated methods consistent with the method described herein.
FIG. 3 is a schematic diagram of a first syringe pump and a first valve system for performing an online, automated method described herein.
FIG. 4 is a schematic diagram of a second syringe pump and a second valve system for performing an online, automated method described herein.
FIG. 5 is a schematic representation of an online, automated method described herein.
FIG. 6 is a schematic representation of an alternate method described herein.
FIG. 7 is a further schematic representation of the online, automated method described herein.
FIG. 8 is a flow chart for a peptide mapping process described herein.
FIG. 9A are graphs showing a calibration curve and a UV determination for a polypeptide using a Protein A capture step.
FIG. 9B is a UV profile used for making a concentration determination for a first eluate and showing that the same amount of protein was captured for the second eluate, which was then used in for protein digestion.
FIG. 10A is a graph showing the correlation between the % glycosylation for a sample IgG1 monoclonal antibody determined using an online peptide mapping method described herein and a glycan release (Oligo 2-AB) method.
FIG. 10B is a graph showing the % quantification of mannose monitored during cell culture over time (Days 7, 10 and 14) using an online peptide mapping method described herein. Replicates of each time point show a constant level of % quantification.
FIG. 11A is a graph showing a direct linear relationship between G0F detection using an online peptide mapping method and a glycan release (Oligo 2-AB) method.
FIG. 11B is a graph showing a direct linear relationship between G1F detection using an online peptide mapping method and a glycan release (Oligo 2-AB) method.
FIG. 11C is a graph showing a direct linear relationship between Man5 detection using an online peptide mapping method and a glycan release (Oligo 2-AB) method.
FIG. 11D is a graph showing a direct linear relationship between G0F-GN detection using an online peptide mapping method and a glycan release (Oligo 2-AB) method.
FIG. 12A is a graph showing the level of oxidation at a particular methionine residue over time during cell culture as determined using an online peptide mapping method described herein.
FIG. 12B is a graph showing the level of N-terminal cyclization over time during cell culture as determined using an online peptide mapping method described herein.

### DETAILED DESCRIPTION OF THE INVENTION

The present invention relates to methods and systems for detecting, identifying or monitoring a post-translational modification (PTM) of a recombinantly produced polypeptide, and in particular, to an online method or system for real-time monitoring of a PTM of a recombinantly produced protein during cell culture.

### A. Definitions

Unless otherwise defined, scientific and technical terms used herein shall have the meanings that are commonly understood by those of ordinary skill in the art. Further, unless otherwise required by context, singular terms shall include pluralities and plural terms shall include the singular, for example, "a" or "an", include pluralities, e.g., "one or more" or "at least one" and the term "or" can mean "and/or", unless stated otherwise. The terms "including," "includes" and "included", are not limiting. Ranges provided herein, of any type, include all values within a particular range described and values about an endpoint for a particular range.

As used herein, the term "about" is used to modify, for example, the quantity of an ingredient in a composition, concentration, volume, process temperature, process time, yield, flow rate, pressure, and ranges thereof, employed in describing the invention. The term "about" refers to variation in the numerical quantity that can occur, for example, through typical measuring and handling procedures used for making compounds, compositions, concentrates or formulations; through inadvertent error in these procedures; through differences in the manufacture, source, or purity of starting materials or ingredients used to carry out the methods, and other similar considerations. The term "about" also encompasses amounts that differ due to aging of a formulation with a particular initial concentration or mixture and amounts that differ due to mixing or processing a formulation with a particular initial concentration or mixture. Where modified by the term "about," the claims appended hereto include such equivalents.

Generally, nomenclatures used in connection with, and techniques of, cell and tissue culture, molecular biology, and protein and oligo- or polynucleotide chemistry and hybridization described herein are those well-known and commonly used in the art. Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

As used herein, the term "polypeptide" refers to a molecule of any length that includes two or more amino acid residues joined to each other by a peptide bond. As used herein, the term "peptide" refers to a polypeptide that includes from about 2 to about 50 amino acids joined to each other by peptide bonds. The term "protein" refers to a polypeptide that includes more than about 100 amino acid residues, or more than about 1000 amino acid residues joined to each other by peptide bonds. The sequence of amino acids in a polypeptide is referred to as the primary structure. In one aspect, the protein has a secondary structure, for example, due to hydrogen bond formation between an oxygen atom of a first amino acid and a nitrogen atom of second amino acid resulting in a two-dimensional alpha-helix or a beta-pleated sheet. In one aspect, the protein has a three-dimensional tertiary structure, for example, due to formation of one or more interchain disulfide (S-S) bonds or other interactions between amino acid R groups, including, but not limited to, hydrogen bonds, ionic bonds, covalent bonds, and hydrophobic interactions. In one aspect, the protein has a quaternary structure, for example, due to the presence of more than one polypeptide chain, for example, an antibody. In one aspect, the peptide does not have a secondary, tertiary, or quaternary structure.

The term "protein" can refer to antibodies and other non-antibody proteins including, but not limited to enzymes, receptors, ligands, secreted proteins, fusion proteins or fragments thereof. Proteins can be of scientific or commercial interest, and can include, but are not limited to therapeutic proteins. The term "therapeutic protein" refers to a protein, that has one or more biological activities that is useful for treating, preventing or ameliorating a disorder or disease.

As used herein, the terms "antibody" and "immunoglobulin" can be used interchangeably and refer to a protein or group of polypeptides that include at least one binding domain that is formed from the folding of polypeptide chain(s) having three-dimensional binding spaces with internal surface shapes and charge distributions complementary to the features of an antigenic determinant of an antigen. A conventional or naturally-occurring antibody typically has a tetrameric form, with two pairs of polypeptide chains, each pair having one "light" and one "heavy" chain. The variable regions of each light/heavy chain pair form an antibody binding site. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (VH) followed by a number of constant domains (CH). Each light chain has a variable domain at one end (VL) and a constant domain (CL) at its other end, wherein the constant domain of the light chain is aligned with the first constant domain of the heavy chain, and the light chain variable domain is aligned with the variable domain of the heavy chain. Light chains are classified as either *lambda* chains or *kappa* chains based on the amino acid sequence of the light chain constant region. Antibodies can be of any isotype (e.g., IgG, IgE, IgM, IgD, IgA and IgY), subisotype (e.g., IgG1, IgG2, IgG3, IgG4, IgA1 and IgA2) or allotype (e.g., Gm, e.g., G1m(f, z, a or x), G2m(n), G3m(g, b, or c), Am, Em, and Km(1, 2 or 3)) and include, but are not limited to, monoclonal antibodies (including full-length monoclonal antibodies), polyclonal antibodies, multispecific antibodies formed from at least two different epitope binding fragments (e.g., bispecific antibodies), CDR-grafted, human antibodies, humanized antibodies, camelised antibodies, chimeric antibodies, anti-idiotypic (anti-Id) antibodies, intrabodies, and desirable antigen binding fragments thereof, and can include recombinantly produced antibody fragments.

The terms "antigen-binding fragment" or "immunologically active fragment" refer to fragments of an antibody that contain at least one antigen-binding site and retain the ability to specifically bind to an antigen. Examples of antibody fragments that can be recombinantly produced include, but are not limited to, antibody fragments that include variable heavy- and light-chain domains, such as single-chain Fvs (scFv), single-chain antibodies, Fab fragments, Fab' fragments, F(ab')₂ fragments. Antibody fragments can also include epitope-binding fragments or derivatives of any of the antibodies enumerated above.

The term "recombinant" refers to a biological material, for example, a nucleic acid or protein, that has been artificially or synthetically (i.e., non-naturally) altered or produced by human intervention, for example, using recombinant DNA processes, including, for example, proteins expressed using a recombinant expression vector transfected into a host cell.

The term "cell culture" refers to methods and techniques used to generate and maintain a population of host cells capable of producing a biomolecule of interest, for example, a protein of interest, as well as methods for producing and collecting the biomolecule of interest. During "cell culture" the protein can be produced intracellularly, in the periplasmic space, or directly secreted into the medium. In one aspect, the cell culture can include mammalian host cells. Suitable culture conditions for mammalian cells are known in the art. See e.g. Animal cell culture: A Practical Approach, D. Rickwood, ed., Oxford University Press, New York (1992). Mammalian host cells may be cultured in suspension or while attached to a solid substrate, for example, in a fluidized bed bioreactor, hollow fiber bioreactor, roller bottle, shake flask, or stirred tank bioreactor, and with or without microcarriers.

"Cell culture media" or "culture media" refers to a liquid or gel that includes an appropriate source of energy and nutrients necessary for the survival and growth of cells in a cell culture. A typical cell culture media includes amino acids; an energy source, usually in the form of a carbohydrate such as glucose; vitamins; free fatty acids; buffers; salts; and trace essential elements. Cell culture media may also be supplemented with additional or increased concentrations of components such as amino acids, salts, sugars, vitamins, hormones, growth factors, buffers, antibiotics, lipids, or trace elements, depending on the requirements of the cells to be cultured and/or the desired cell culture parameters.

"Conditioned media" refers to culture media containing a biomolecule of interest that is released or secreted by cells into a cell culture media.

"Clarified cell culture media" refers to culture media from which host cells and/or cellular debris has been removed, for example, by centrifugation, microfiltration, tangential flow filtration, alternating tangential flow filtration and/or depth filtration. In one aspect, the clarified cell culture media includes a cell culture supernatant.

"Bioreactor" refers to a receptacle designed for controlled growth of a cell culture. The term bioreactor can refer to any scale cell culture, from individual flasks and shaker flasks or wave bags, to one-liter bioreactors, and up to large-scale industrial bioreactors. In one aspect, the bioreactor is a small-scale bioreactor, for example, having a volume from about 125 ml to about 500 ml. In one aspect, bioreactor is a large-scale bioreactor, for example, having a volume from about 100 liters to about 20,000 liters or more. A variety of bioreactors are known and include, but are not limited to, stirred-stank, fixed-bed, fluidized bed, bubble column, and airlift bioreactors. Large-scale or commercial-scale cell cultures, for example, for the production of a therapeutic protein, can be maintained for days, or even weeks. During this time, the culture can be supplemented with a concentrated feed medium that contains components, such as nutrients and amino acids, which are consumed during the course of the cell culture. In one aspect, the media in the bioreactor is not supplemented during the cell culture process in a batch process. In one aspect, the media in the bioreactor is supplemented with additional components, for example, nutrients or cell culture media, during the cell culture process in a fed-batch bioreactor. In one aspect, the media is continually added to the bioreactor and spent media and product are removed throughout the cell culture process in a perfusion bioreactor.

"Host cell" or "recombinant host cell" is a cell into which an expression vector has been introduced. The term "host cell" refers not only to the particular cell, but also to the progeny of such cell. Because certain modifications may occur in succeeding generations due to either mutation or environmental influences, such progeny may not, in fact, be identical to the parent cell, but are still included within the scope of the term "host cell". Host cells can include prokaryotic and eukaryotic cells. In one aspect, the host cell is a mammalian host cell, for example, Chinese hamster ovary cells (CHO), human embryonic kidney cells (e.g., HEK 293), monkey kidney cells (e.g., COS), murine myeloma cells (e.g., NS0 or SP2), or human primary embryonic retinal cells (PER.C6).

"Critical quality attributes" (CQA) are chemical, physical, or biological properties of a therapeutic protein that may impact product safety and/or efficacy. CQA can include product-specific variants including, for example, size and sequence variants, variants due to aggregation or fragmentation, and charge variants. Other variants include post-translational modifications (PTM) such as glycosylation, oxidation, deamidation, isomerization, N-terminal processing, C-terminal processing. CQA also include process-related impurities, including, but not limited to, host cell protein, DNA, leachables and other raw materials.

"Post-translational modification" (PTM) refers to a change in a polypeptide as a result of a covalent addition of a functional group to the polypeptide chain or cleavage of a peptide bond within the polypeptide chain and include, but are not limited to, glycosylation, oxidation, deamidation, isomerization, glycation, N-terminal leader sequence, N-terminal cyclization, C-terminus lysine retention, amide formation or a combination thereof. In one aspect, glycosylation includes N-linked glycosylation, O-linked glycosylation or a combination thereof.

"Glycoprotein" refers to protein that includes one or more carbohydrate or saccharide moieties appended along the length of the polypeptide chain. "Glycopeptide" refers to a peptide that includes one or more carbohydrate or saccharide moieties appended along the length of the polypeptide chain.

"Glycoform" refers to an isoform of a protein that differs in glycosylation profile, i.e., the number and/or type of attached glycans.

"Glycan" refers to an oligosaccharide attached to a polypeptide chain and includes N-linked and O-linked glycans. N-linked glycans (also called N-glycans) are attached to the polypeptide chain at an amide nitrogen of an Asparagine (Asp) or Arginine (Arg) residue via an N-glycosidic bond. N-linked glycosylation occurs at a consensus sequence (Asn-X-Ser/Thr, where X is any amino acid except proline). O-linked glycans (or O-glycans) are attached to the polypeptide chain at Serine (Ser) or Threonine (Thr) residues via an α-O-glycosidic bond. There is no general consensus sequence for O-linked glycosylation. Both N- and O-linked glycosylation occur in the endoplasmic reticulum (ER) and Golgi in a reaction pathway that includes step-wise addition and/or removal of individual monosaccharides. There are many types of glycans which can be categorized by the presence of absence of different terminal sugars, including, for example, agalactosyl glycans with 0 terminal galactose moieties (e.g., G0, G0-GlcNAc, G0F, G0F-GlcNAc), glycans with 1 terminal galactose moiety (e.g., G1, G1F, G1F-GlcNAc), and glycans with 2 terminal galactose moieties (e.g., G2F, G2F +Sialic Acid). While some glycosylation patterns can be beneficial to protein function, others may be detrimental.

"Process Analytical Technologies" (PAT) refers to a system for designing, analyzing, and controlling manufacturing through timely measurements, for example, during processing, of critical quality and performance attributes of raw and in-process materials and processes.

"Online" refers to an analytical process in which automatic sampling of a process solution is conducted without human intervention. "Inline" refers to an analytical process in which a sensor is placed within a stream of flowing material to conduct the analysis. "At-line" refers to a process in which a sample is transferred from the process to an analytical instrument in close physical proximity to the process and returns analytical result in a relatively short period of time (i.e., hours). "Offline" refers to a process in which a sample is removed from the process and analyzed at a different location, wherein the analytical results are not returned within a relatively short period of time (e.g., days). Both offline and at-line analyses require human intervention in which samples are manually taken from the system, followed by sample preparation and analysis. Online and inline analyses can be fully-automated and performed without human intervention.

"Real-time" means that an analytical process is performed without a significant time delay, for example, within a short enough period of time that the information obtained from the analytical process can be applied in making decisions regarding optimizing process conditions, for example, in which cell culture conditions in a bioreactor can be optimized while the cell culture from which the sample was taken is still in progress. In one aspect, the results of the analytical process, including the protein A capture step and the protein concentration determination, are available within less than about 5 hours, about 4 hours or about 3 hours. In one aspect, the results of the analytical process are available within less than about 2.5 hours. In contrast, in an offline process the results of the analytical processes generally take at least 1 day and up to 2 days.

"Automated" refers to a process that is performed without human invention. In one aspect, the automated process is performed by one or more instruments. In one aspect, the automated process includes a process in which sample preparation is online and automated. In one aspect, the online, automated sample preparation process is performed in a fluid injection analyzer (FIA). In one aspect, the fluid injection analyzer is a sequential injection analyzer (SIA). In one aspect, the fluid injection analyzer is a direct injection analyzer (DIA). In one aspect, the automated process includes an automated analytical instrument, for example, an automated peptide mapping unit. The term "automated" does not include offline automated processes, for example, processes using a liquid handling system such as a Hamilton liquid handling system (Hamilton Company, Reno, NV, U.S.A.) or a TECAN liquid handling system (TECAN Group Ltd., Männedorf, Swizerland).

"Predetermined" refers to something that was determined prior to initiation of a process, for example, prior to initiation of the method described herein. A "predetermined calibration curve," for example, for use in determining protein concentration, refers to a calibration curve that is generated using representative samples with known protein concentrations. For example, a calibration curve can be generated by obtaining a sample with a known concentration, performing serial dilutions, for example, 10-fold serial dilutions, to obtain from about 3 to about 10 samples of known concentration. In one aspect, the UV absorbance of the serially diluted samples is then determined, along with the UV absorbance of a blank that includes all reagents except for the protein. The calibration curve is generated by plotting the UV absorbance against the known protein concentrations of the samples. In one aspect, a "predetermined elution time" is determined by loading a known amount of a representative sample onto a column and determining the amount of time required to elute the sample from the column. In one aspect, the elution time refers to the amount of time elapsed between the time at which the sample is loaded onto the column and the time at which sample is no longer eluted from the column when the elution reagent is applied at a known rate. In one aspect, the elution time refers to the amount of time elapsed between the time at which the sample is loaded onto the column and the time at which the maximum concentration of sample is eluted from the column when the elution reagent is applied at a known rate. In one aspect, the elution time can refer to the volume of elution reagent at a known flow rate that is required to elute all sample from the column. A "predetermined" post-translational modification refers to a known post-translational modification of a protein of interest that is identified prior to the method described herein, wherein the method is used to identify and/or quantify the predetermined post-translational modification.

"Concurrently" when used in connection with method steps described herein, refers to a method in which the steps are performed substantially at the same time. Actions that are performed "substantially at the same time" can mean that the actions are performed simultaneously, but can also include situations when the two actions are performed within a short time of each other, for example, within about 30 minutes of each other, or within about 15, about 10, about 5, about 4, about 3, about 2 or about 1 minute of each other. In one aspect, method steps are performed "concurrently" when the execution of at least a portion of one method step overlaps in time with the execution of a portion of another method step. "Concurrently" does not require exact simultaneous activity. For example, it is not required that all method steps begin or end at the same time. In one aspect, "concurrently" can mean that all reagents necessary for the method steps are combined in the same reaction mixture such that the reactions occur in the same reaction volume and/or during the same incubation period.

"Substantially the same" or "substantially match" when used with respect to two process parameters, including, but not limited to, quantity, volume, time, temperature, pressure, concentration, and flow rate, means the quantity or quantification of the two process parameters is functionally equivalent, for example, the difference between the two process parameters is less than about 10%, about 5%, about 2%, about 1%, about 0.5% or about 0.1%.

"Flow injection analysis" (FIA) refers to an analytical technique in which microliter volumes of a sample are injected into and transported by a carrier stream for automated sample processing. Flow injection analysis may be performed by a flow injection analyzer (FIA), which, as used herein, may include or encompass a sequential injection analyzer (SIA) or a direct injection analyzer (DIA). In one aspect, the flow injection analyzer uses a carrier stream at a constant flow to transport the sample through the analyzer. In one aspect, the flow injection analyzer includes a bi-directional pump, for example, a syringe pump, that can reverse or stop the flow of the carrier stream to mix a sample with reagent or to assay sample components.

As used herein, "in fluid communication" means that a fluid, for example, a liquid, can flow from one component, apparatus or device to another component, apparatus or device in a system. Flow may be achieved by way of one or more intermediate components, apparatuses, or devices and may or may not be selectively interruptible, for example, by a valve.

As used herein, "coupled" means that two or more components, apparatuses or devices are joined or otherwise attached, movably or fixedly, by one or more intermediate components, apparatus or devices.

As used herein, "upstream" and "downstream" refer to relative locations with respect to the flow direction of a carrier stream in a system. The term "upstream" refers to a system component or location that is closer to the input of a system relative to another component or location. The term "downstream" refers to a system component or location that is closer to the outlet of a system relative to another component or location.

"Column chromatography" refers to a process in which components in a liquid sample are separated based on the differential adsorption of components in the sample as the mobile phase of the sample flows over a stationary phase.

"Affinity chromatography" refers to chromatographic process in which a biomolecule of interest, for example, a protein, in a mobile phase is retained by the stationary phase of the chromatographic column based on a specific, reversible binding interaction between the component of interest and a compound immobilized on the chromatographic matrix to effect chromatographic separation, including, but not limited to, interactions between a receptor and a ligand, an enzyme and a substrate or an antigen and an antibody, rather than a general property of the protein such as isoelectric point, hydrophobicity, or size.

"Protein A chromatography" refers to an affinity chromatography process in which an antibody is purified from a sample based on the affinity between the Fc portion of the antibody and an IgG binding domain of Protein A immobilized on the stationary phase.

"Liquid chromatography" (LC) refers to a process in which one or more components of a liquid sample are selectively retained as the sample is passed through a column. The retention of one or more components in the sample results from the distribution of the components between a stationary phase and a mobile phase. Liquid chromatography includes, but is not limited to, reverse phase liquid chromatography (RPLC), high performance liquid chromatography (HPLC), and ultra-high performance liquid chromatography (UHPLC).

"Mass spectrometry" (MS) refers to an analytical technique in which a compound is detected, identified and/or measured based on its mass to charge ratio (m/z). MS generally involves ionization of the compounds, separation of the ionized molecules according to their mass-to-charge ratio, detection of the ionized molecules and generation of a mass spectrum. A known method for protein ionization is Electrospray ionization (ESI). "Mass spectrometer" refers to an analytical instrument capable of detecting, identifying and/or quantifying a compound based on its mass to charge ratio. A mass spectrometer generally includes three parts: an ion source, a mass analyzer and a detector.

As used herein "liquid chromatography/ mass spectrometry" (LC/MS) refers to a method in which components in a sample, for example, peptides in a peptide mixture, are separated by liquid chromatography and then ionized and characterized by their mass to charge ratio.

"Tandem mass spectrometry" (MS/MS) refers to a technique in which a compound is detected, identified and/or measured using a multi-stage process that includes more than one step of mass spectrometry, in which fragmentation is performed between the mass spectrometry processes.

### B. Overview

Peptide mapping is an important analytical tool used during the development of cell culture processes, as well as during manufacturing of biomolecules using cell culture processes. In one aspect, peptide mapping can be used to confirm the sequence of biomolecule. In one aspect, peptide mapping can be used to identify and quantify a post-translational modification (PTM) of a peptide produced in cell culture. In one aspect, peptide mapping can be used for multi-attribute monitoring (MAM) of Critical Quality Attributes (CQAs).

Many methods have been developed to analyze PTMs. One of the most widely used approaches includes peptide mapping, includes liquid chromatography (LC)/mass spectrometry (MS). In one method, glycans are chemically labelled, for example using 2-aminobenzamide (2-AB) or 2-aminobenzoic acid (2-AA), enzymatically or chemically cleaved from the glycoprotein, purified and analyzed by liquid chromatography (LC) using a fluorescence detector. This process is referred to as a "fluorescent labelled glycan release" method. Because the glycans are removed from the protein, the glycan release is unable to provide site-specific information. In another method, the glycoprotein is digested into peptides using an enzyme, such as trypsin, prior to LC/MS analysis. Because the glycans remain on the peptides, site-specific information about PTM, for example, glycosylation can be obtained for the peptides. In one aspect, the peptides identified by the mass spectrometer are used as surrogate representatives of the intact protein obtained from the cell culture media.

Provided herein is an automated online system and method for analyzing in-process samples for real-time monitoring of product quality and process controls during cell culture. In contrast to a conventional peptide mapping approach, which can take more than 2 days, for example, up to about 3 or about 4 days to complete, the online peptide mapping method described herein provides a fully automated process that can provide information about glycosylation trending, post-translational modifications and other site-specific information in less than a day, even as little as 2.5 hours. In one aspect, the system described herein provides a fully automated method for sample preparation and peptide mapping analysis. In one aspect, the method provides a fully automated system for protein capture, protein concentration determination and protein digestion. In one aspect, the online peptide mapping method described herein is used to detect one or more known post-translational modifications (PTM) associated with a protein of interest. In one aspect, the online peptide mapping method described herein is used to detect one or more predetermined post-translational modifications associated with a protein of interest. Advantageously, site-specific information regarding PTM can be obtained using the method described herein.

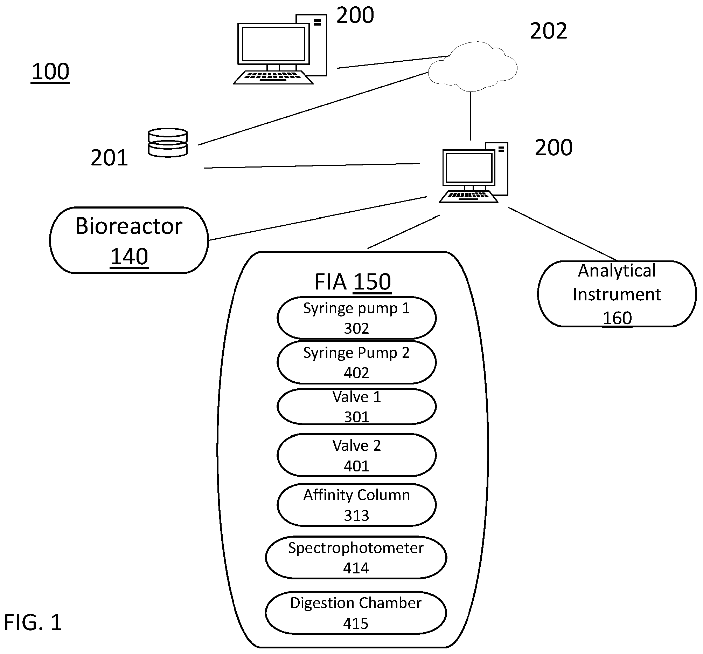

FIGS. 1-4 provide an overview of a system configured for executing or performing the automated methods as described herein.

FIG. 1 is a schematic diagram of a system configured to provide process implementation of automated methods consistent with an automated method and system described herein. FIG. 1 illustrates an automated process control system 100 configured to perform automated processing methods as described herein. In FIG. 1, one aspect of a network environment is depicted. The network environment may include one or more process control devices 200 in communication with one or more of a bioreactor 140, a flow injection analyzer (FIA) 150, one or more data retention systems 201, one or more other process control devices 200, and one or more analytical instruments 160, via one or more networks 202.

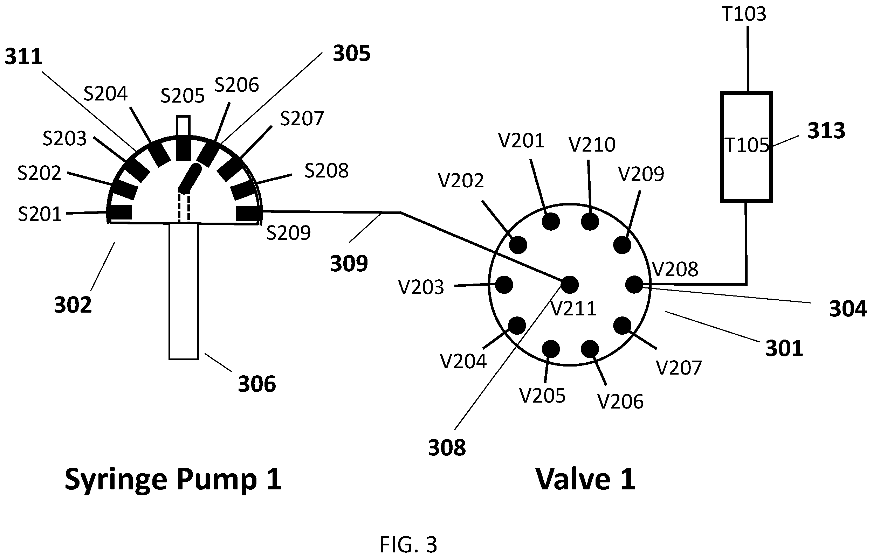

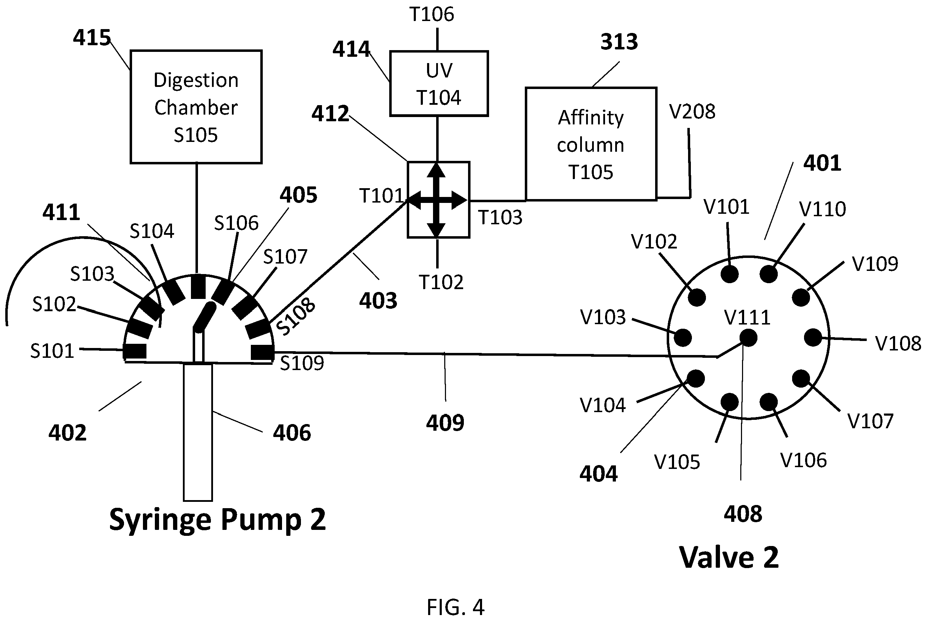

As shown in FIG. 3 and 4, the FIA 150 may include at least a first syringe pump 302, a second syringe pump 402, a first valve 301, and a second valve 401. Further, the FIA 150 may include at least one affinity column 313, at least one spectrophotometer 414, and at least one digestion chamber 415. In addition, the FIA 150 may include further components such as tubing, T-junctions, valves, and other components necessary to route fluids to appropriate destinations. Additionally, the FIA 150 may contain, include, or otherwise be connected to supply sources for samples, reagents, reactants, water, reductants, enzymes, buffers, etc., drain sinks for the disposal of waste. The FIA 150 may be configured as a flow injection analyzer and/or with any or all of the functionality of a sequential injection analyzer (SIA) or a direct injection analyzer (DIA).

The automated process control system 100 may further include one or more bioreactors 140. Samples for processing via the automated process control system 100 may be obtained from the one or more bioreactors 140, either manually or through an automated feed process to the FIA 150. Further description of bioreactors 140 consistent with method described herein are discussed below.

The one or more analytical instruments of the automated process control system 100 may include a peptide mapping system with one or more mass analyzers.

Data and information stored by the automated process control system 100 may include the following information. As used herein, "automated process control system data" refers to any and all data that may be recorded and stored on or in a memory associated with an automated process control system 100. Automated process control system data may be stored in any suitable data format, and may be sortable by production batch, production date, or any other suitable parameter. "Process information," as used herein, refers to information about variables and parameters of sample processing methods and techniques, including, but not limited to, timing, temperature, additive amounts, calibration coefficients, flow rates, and models, etc. "Production information," as used herein, refers to information about processed samples, previous sample runs, etc. Each of the above-described data and/or information may be accessed in near-real time by process control devices 200 discussed herein. Data and process information associated with the automated process control system 100 may be stored, for example, in a data retention system 201, as discussed below, and/or within memories of the one or more process control devices 200.

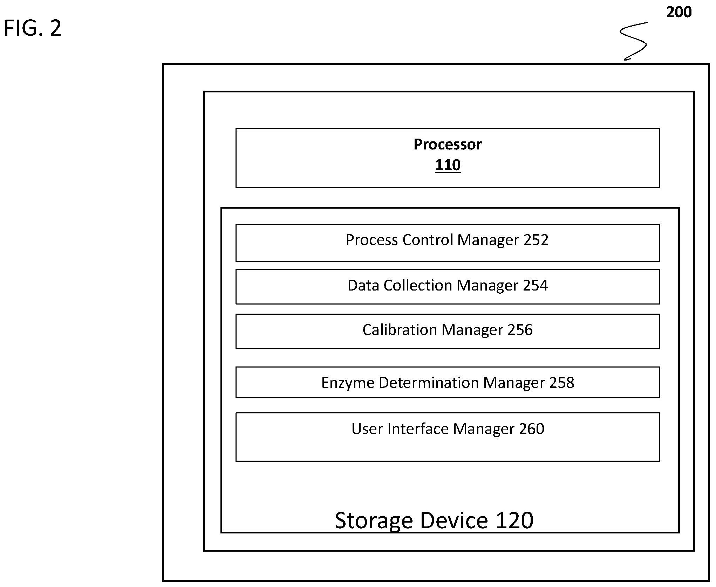

The process control device 200 may be configured as a server (e.g., having one or more server blades, processors, etc.), a personal computer (e.g., a desktop computer, a laptop computer, etc.), a smartphone, a tablet computing device, a computing system integrated with the FIA 150, and/or other device that can be programmed to interface with the bioreactor 140, the FIA 150, and/or the mass analyzer 160. In one aspect, any, or all of the functionality of the process control device 200 may be performed as part of a cloud computing platform. The process control device 200 is further discussed below with respect to FIG. 2.

The network environment depicted in FIG. 1 represents an example of an automated process control system 100 and process control devices 200 configured to provide automated methods as described herein. Although depicted as connected via network 202, any suitable series of individual or network connections may be employed to permit the process control device 200 to control the bioreactor 140, the FIA 150, and the analytical instrument 160, and access required resources such as various data retention systems 201.

The network 202 may be connected via wired or wireless links. Wired links may include Digital Subscriber Line (DSL), coaxial cable lines, ethernet, or optical fiber lines. Wireless links may include Bluetooth^{®}, Bluetooth Low Energy (BLE), ANT/ANT+, ZigBee, Z-Wave, Thread, Wi-Fi^{®}, Worldwide Interoperability for Microwave Access (WiMAX^{®}), mobile WiMAX^{®}, WiMAX^{®}-Advanced, NFC, SigFox, LoRa, Random Phase Multiple Access (RPMA), Weightless-N/P/W, an infrared channel or a satellite band. The wireless links may also include any cellular network standards to communicate among mobile devices, including standards that qualify as 2G, 3G, 4G, or 5G. Wireless standards may use various channel access methods, e.g., FDMA, TDMA, CDMA, or SDMA. In one aspect, different types of data may be transmitted via different links and standards. In another aspect, the same types of data may be transmitted via different links and standards. Network communications may be conducted via any suitable protocol, including, e.g., http, tcp/ip, udp, ethernet, ATM, etc.

The network 202 may be any type and/or form of network. The geographical scope of the network may vary widely and the network 202 can be a body area network (BAN), a personal area network (PAN), a local-area network (LAN), e.g., Intranet, a metropolitan area network (MAN), a wide area network (WAN), or the Internet. The topology of the network 202 may be of any form and may include, e.g., any of the following: point-to-point, bus, star, ring, mesh, or tree. The network 202 may be of any such network topology as known to those ordinarily skilled in the art capable of supporting the operations described herein. The network 202 may utilize different techniques and layers or stacks of protocols, including, e.g., the Ethernet protocol, the internet protocol suite (TCP/IP), the ATM (Asynchronous Transfer Mode) technique, the SONET (Synchronous Optical Networking) protocol, or the SDH (Synchronous Digital Hierarchy) protocol. The TCP/IP internet protocol suite may include application layer, transport layer, internet layer (including, e.g., IPv4 and IPv4), or the link layer. The network 202 may be a type of broadcast network, a telecommunications network, a data communication network, or a computer network.

The data retention systems 201 may include any type of computer readable storage medium (or media) and/or a computer readable storage device. Such computer readable storage medium or device may be configured to store and provide access to data. Examples of a computer readable storage medium or device may include, but are not limited to, an electronic storage device, a magnetic storage device, an optical storage device, an electromagnetic storage device, a semiconductor storage device, or any suitable combination thereof, for example, such as a computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick.

FIG. 2 is a schematic diagram of a computer system or process control device configured to provide process control of automated methods consistent with method described herein. The process control device 200 includes one or more processors 110 (also interchangeably referred to herein as processors 110, processor(s) 110, or processor 110 for convenience), one or more storage device(s) 120, and/or other components. In another aspect, the functionality of the processor may be performed by hardware (e.g., through the use of an application specific integrated circuit ("ASIC"), a programmable gate array ("PGA"), a field programmable gate array ("FPGA"), etc.), or any combination of hardware and software. The storage device 120 includes any type of non-transitory computer readable storage medium (or media) and/or non-transitory computer readable storage device. Such computer readable storage media or devices may store computer readable program instructions for causing a processor to carry out one or more methodologies described here. Examples of the computer readable storage medium or device may include, but is not limited to an electronic storage device, a magnetic storage device, an optical storage device, an electromagnetic storage device, a semiconductor storage device, or any suitable combination thereof, for example, such as a computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, but not limited to only those examples.

The processor 110 is programmed by one or more computer program instructions stored on the storage device 120 representing software protocols. For example, the processor 110 is programmed by a process control manager 252, a data collection manager 254, a calibration manager 256, an enzyme determination manager 258, and a user interface manager 260. It will be understood that the functionality of the various managers as discussed herein is representative and not limiting. Additionally, the storage device 120 may act as a data retention system 201 to provide data storage. As used herein, for convenience, the various "managers" will be described as performing operations, when, in fact, the managers program the processor 110 (and therefore the process control device 200) to perform the operation.

The various components of the process control device 200 work in concert to provide control of one or more FIAS 150, and one or more analytical instruments 160 and to provide an interface for a user or other system to interface with these systems.

The process control manager 252 is a software protocol operating on the automated process control system 100. The process control manager 252 is configured to provide one or more control signals to the FIA 150, and the analytical instrument 160. The control signals provided by the process control manager 254 are configured to cause an adjustment of one or more process parameters of the FIA 150, and the analytical instrument 160. As used herein and discussed above, "process parameters" refers to any parameter or variable of the production process that can be adjusted by a user through the process control device 200. Process parameters include, but are not limited to, routing, flow rates, temperature, incubation periods, reagent additions, timing of various process steps, etc. Determination of the control signals may be based on process information or production information received by the data collection manager 254, as discussed herein.

Control signals provided by the process control manager 252 may be used to initiate, control, and/or update any process that the FIA 150, and the analytical instrument 160 described herein are capable of. For example, the process control manager 252 is configured to control, via automated control of the various actuation means of the FIA 150, the flow, routing, and timing of all samples, fluids, reagents, temperatures, agitation, incubation periods, etc. throughout the FIA 150 during an automated process.

In one aspect, the process control manager 252 provides a process monitoring function. The process control manager 252 may be configured to access any and all information measured, produced, and/or stored by the process control device 200. The process control manager 254 may further be configured to provide any of such information to a user via the user interface manager 260.

In another aspect, the process control manager 252 may be configured to communicate with and/or otherwise interact with the calibration manager 256 and the enzyme determination manager 258. The calibration manager 256 and the enzyme determination manager 258 are discussed in greater detail below.

In one aspect, the process control manager 252 is configured to control the operation of the FIA 150 and the analytical instrument160, as further discussed below with specific respect to automated methods described herein.

In one aspect, the process control manager 252 controls flow of a sample through the affinity column 313 for a predetermined elution time, as discussed in greater detail below. In another aspect, the process control manager 252 controls flow of a sample through the affinity column 313 to achieve a predetermined volume of eluate as an output. The process control manager 252 may control the sample flow to achieve a predetermined volume of eluate in a predetermined elution time. The process control manager 252 is further configured to control flow of eluate from an output of the affinity column 313 to the spectrophotometer 414 to enable determination and/or measurement of a protein concentration of the eluate by the data collection manager 254, as discussed below. In one aspect, the process control manager 252 is configured to control flow of eluate from an output of the affinity column 313 to the digestion chamber to enable digestion of the protein by addition of an amount of digestion reagent determined by the process control manager 252, as discussed below. In one aspect, the process control manager 252 is configured to control the incubation time and temperature of a sample in the digestion chamber 415.

The data collection manager 254 is a software protocol operating on the process control device 200. The data collection manager 254 is configured to access one or more of the FIA 150, the analytical instrument 160, the affinity column 155, the spectrophotometer 156 and the digestion chamber 415 to gather and/or collect data and information associated with the automated processes described herein. Automated system data may include, for example, production information, which may be obtained in near real time, archived data, and/or data extracts, as well as process information and process parameter information and any other information or data generated or measured by the FIA 150, and the analytical instrument 160. The data collection manager 254 is further configured to access one or more data retention systems 201 to store and/or receive automated processing system data stored in the data retention system 201.

The data collection manager 254 is configured to interface with the spectrophotometer 156 to perform protein concentration measurements/determinations on an eluate produced or output by the affinity column 313.

The calibration manager 256 is a software protocol operating on the process control device 200. The calibration manager 256 is configured to receive and manage calibration information related to determining protein concentrations based on UV absorbance measurements made by the spectrophotometer 414. The calibration manager 256 is configured to communicate and cooperate with the process control manager 252 and the data collection manager 254 to collect raw data from the spectrophotometer 414. The calibration information stored and managed by the calibration manager 256 may be applied by the process control manager 252 to the raw data (e.g., UV absorbance data) collected by the data collection manager 254 to determine protein concentrations in a sample eluate.

The enzyme determination manager 258 is a software protocol operating on the process control device 200. The enzyme determination manager 258 is configured to determine an amount of enzyme to be added to a sample during an automated processing procedure. As described in greater detail below, during an automated processing procedure, a first sample may be eluted via the affinity column 313 for a controlled predetermined elution time by the process control manager 252 to obtain a sample eluate. The data collection manager 254 operates to obtain protein concentration measurements, which, combined with calibration information, may be used by the process control manager 252 to determine protein concentration levels in the eluted sample. The enzyme determination manager 258 may use the determined protein concentration levels to determine appropriate amounts of digestion enzyme to be added to a sample digestion step.

In operation, the enzyme determination manager 258 may communicate and cooperate with the process control manager 252 to provide the determined digestive enzyme information regarding enzyme additive amounts for the second sample as determined by the protein concentration determination of the first sample. As discussed above, the process control manager 252 is configured to precisely control the timing and volume of sample flow through the affinity column 313. By applying the same predetermined timing and volume processing parameters to a first sample and a second sample processed by the affinity column 313, the process control manager 252 may be able to achieve a first sample eluate and a second sample eluate having substantially similar concentration values. Accordingly, measurements of protein concentration performed on a first sample eluate, as discussed above, may be applied by the enzyme determination manager 258 to determine appropriate amounts of digestive enzyme to be added to the second sample eluate.

The user interface manager 260 is a software protocol operating on the process control device 200. The user interface manager 260 is configured to provide a user interface to allow user interaction with the process control device 200. The user interface manager 260 is configured to receive input from any user input source, including but not limited to touchscreens, keyboards, mice, controllers, joysticks, voice control. The user interface manager 260 is configured to provide a user interface, such as a text-based user interface, a graphical user interface, or any other suitable user interface. The user interface manager 260 may be configured to provide different user interface services depending on a type of client device. For example, a laptop or desktop computer may be provided with a user interface including a full suite of interface options, while a smartphone or tablet may be provided with a user interface limited to status updates.

In one aspect, the user interface manager 260 may provide one or more users with access to any or all process and/or production information about the automated process control system 100 via a user interface. The user interface manager 260 may permit a user to perform various tasks on the bioreactor 140, the FIA 150, and the analytical instrument 160. For example, the user interface manager 260 may permit a user to adjust or control one or more process parameters directly.

FIG. 3 is a schematic diagram of a first syringe pump and a first valve system associated with the FIA 150 for performing an online, automated method described herein. In operation, the process control manager 252 of the process control device controls the first valve 301 and the first syringe pump 302 of the FIA 150 to appropriately route and mix fluids during automated process methods described herein. Table 1 below provides an example listing of the various connections to the first valve 301 and the first syringe pump 302.

The first valve 301 includes a plurality of input ports 304 and an output port 308. The terms "input port" and "output port" are used herein to differentiation the ports at either end of the first valve 301 and do not necessarily refer to flow directions associated therewith. The first valve 301 may be, for example, a multi-port rotary valve. The process control manager 252 controls a valve automated selector means (e.g., a stepper motor, servo motor, or other actuator device, not pictured) configured to rotate the valve 301 to provide fluid communication between a selected input port 304 and the output port 308. The output port 308 is connected to the first syringe pump 302 via communication line 309. The first syringe pump 302 includes a port selection device 311 and an actuation syringe 306. The port selection device 311 includes a plurality of syringe pump ports 305 and a syringe pump automated selector means (e.g., a stepper motor, servo motor, or other actuator device, not pictured) configured to manipulate the port selection device 311 to align a selected one of the plurality of syringe pump ports 305 with the actuator syringe 306. The syringe pump automated selector means is controlled by the process control manager 252 to select a specific syringe pump port 305 to arrange in fluid communication with actuator syringe 306.

The actuator syringe 306 is a syringe actuated by a syringe pump actuation means (e.g., a stepper motor, linear drive, servo motor, or other actuator device, not pictured) providing bi-directional pressure to draw in and push out fluids through a selected one of the syringe pump ports 305. The actuator syringe 306 is further configured to mix fluids with a syringe chamber via depression and retraction of a syringe plunger as activated by the syringe pump actuation means. The actuator syringe 306 may be any type of syringe configured to bi-directionally apply pressure to the FIA 150 system to draw fluids in and press fluids out.

Further illustrated in FIG. 3 is the affinity column 313, connected to the first valve 301 by V208 input port 304

In operation, the process control manager 252 may operate the first valve 301 to establish a fluid connection between a selected input port 304 and the output port 308. The process control manager 252 may operate the port selection device 311 to generate a fluid connection between a selected syringe pump port 305 and the actuator syringe 306. The actuator syringe 306 may then be actuated to draw a specific amount of fluid into the actuator syringe 306 via the selected syringe pump port 305. The port selection device 311 may then be operated again by the process control manager 252 to generate fluid communication between the communication line 309 and the syringe pump actuator 306. The actuator syringe 306 may then be operated again to draw fluid from the first valve 301 into the actuator syringe 306. The actuator syringe 306 and port selection device 311 may then be operated further to draw additional fluids into the actuator syringe 306 and/or to move the fluids in the actuator syringe out through one of the syringe pump ports 305.

The process control manager 252 is capable of providing accurate and precise control of the actuator syringe 306 so as to transfer appropriate amounts of fluids through the system of the FIA 150.

FIG. 4 is a schematic diagram of a second syringe pump and a second valve system associated with the FIA 150 for performing an online, automated method described herein. In operation, the process control manager 252 of the process control device controls the second valve 401 and the second syringe pump 402 of the FIA 150 to appropriately route and mix fluids during automated process methods described herein. Table 1 below provides an example listing of the various connections to the second valve 401 and the second syringe pump 402.

The second valve 401 includes a plurality of input ports 404 and an output port 408. The second valve 401 may be, for example, a multi-port rotary valve. The terms "input port" and "output port" are used herein to differentiation the ports at either end of the first valve 301 and do not necessarily refer to flow directions associated therewith. The process control manager 252 controls a valve automated selector means (e.g., a stepper motor, servo motor, or other actuator device, not pictured) configured to rotate the valve 401 to provide fluid communication between a selected input port 404 and the output port 408. The output port 408 is connected to the second syringe pump 402 via communication line 409. The second syringe pump 402 includes a port selection device 411 and an actuation syringe 406. The port selection device 411 includes a plurality of syringe pump ports 405 and a syringe pump automated selector means (e.g., a stepper motor, servo motor, or other actuator device, not pictured) configured to manipulate the port selection device 411 to align a selected one of the plurality of syringe pump ports 405 with the actuator syringe 406. The syringe pump automated selector means is controlled by the process control manager 252 to select a specific syringe pump port 305 to arrange in fluid communication with the valve 301 via the communication line 309.

The actuator syringe 406 is a syringe connected to a syringe pump actuation means (e.g., a stepper motor, linear drive, servo motor, or other actuator device, not pictured) providing bi-directional pressure to draw in and push out fluids through a selected one of the syringe pump ports 405. The actuator syringe 406 is further configured to mix fluids with a syringe chamber via depression and retraction of a syringe plunger as activated by the syringe pump actuation means. The actuator syringe 406 may be any type of syringe configured to bi-directionally apply pressure to the FIA 150 system to draw fluids in and press fluids out.

Further illustrated in FIG. 4 is the affinity column 313, connected at an input end to the first valve 301 and connected at an output end to a multiway junction 412. The multiway junction 412 is further connected to a spectrophotometer 414. Further illustrated in FIG. 4 is a S105 digestion chamber 415, connected to the second syringe pump 402 via a syringe pump port 405.

In operation, the second valve 401 and the second syringe pump 402 operate similarly to the first valve 301 and the first syringe pump 302. The process control manager 252 provides control signals the valve automated selector means, the syringe pump automated selector means, and the syringe pump actuation means to control and arrange the connections of the FIA 150 and to cause/provide fluid fluid flow throughout the FIA 150 in accordance with process and production parameters.

Table 1 below provides an example of the various fluid connections, fluid reservoirs/sources, fluid sinks/outlets/drains, etc., in a FIA 150 consistent with the system described herein.

**Table 1**

| **Syringe Pump 1** | **Valve 1** | **Syringe Pump 2** | **Valve 2** | **Junction 1** |
|---|---|---|---|---|
| S201: Binding buffer | V201: Waste | S101: Water | V101: Waste | T101: From S108 |
| S202: Washing solution 3 | V202: Calibrant 1 | S102: Proteolytic enzyme | V102: Available | T102: Available |
| S203: Available | V203: Elution buffer | S103: Reducing reagent | V103: Available | T103: From Affinity column |
| S204: Available | V204: Calibrant 2 | S104: Available | V104: Digestion buffer | T104: To UV |
| S205: Available | V205: Calibrant 3 | S105: Digestion Chamber | V105: Wash solution 1 | T105: Affinity column |
| S206: Available | V206: Calibrant 4 | S106: Waste | V106: Sample Reservoir | T106: Waste |
| S207: Available | V207: Calibrant 5 | S107: Available | V107: Available | |
| S208: Available | V208: To T105 | S108: Column return | V108: LC system | |
| S209: To V211 | V209: Sample Reservoir 1 | S109: Outlet to Valve 1 | V109: Wash solution 2 | |
| | V210: Sample Reservoir 2 | | V110: Air | |
| | V211: From S209 | | V111: S109 | |

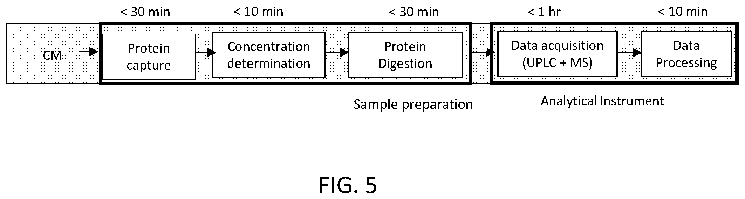

A schematic overview of the online, automated method is provided in FIG. 5, with references to the automated process system 100 as described above. Briefly, clarified cell culture media is delivered to a sample preparation unit that includes a protein capture unit, a protein concentration determination unit, and a protein digestion unit. In one aspect, the protein capture unit includes a chromatography column. In one aspect, the protein capture unit includes an affinity chromatography column (also referred to herein as an affinity column). In one aspect, the protein concentration determination unit includes a spectrophotometer. In one aspect, the protein digestion unit includes a digestion chamber. In one aspect, the protein digestion chamber is the barrel of a syringe pump. In one aspect, the temperature of the digestion chamber can be controlled. In one aspect, the prepared sample is transferred to an analytical instrument. In one aspect, the analytical instrument includes a peptide mapping unit. In one aspect, the peptide mapping unit includes a data acquisition unit and a data processing unit. In one aspect, the data acquisition unit includes a liquid chromatography column (LC) and a mass spectrometer (MS).

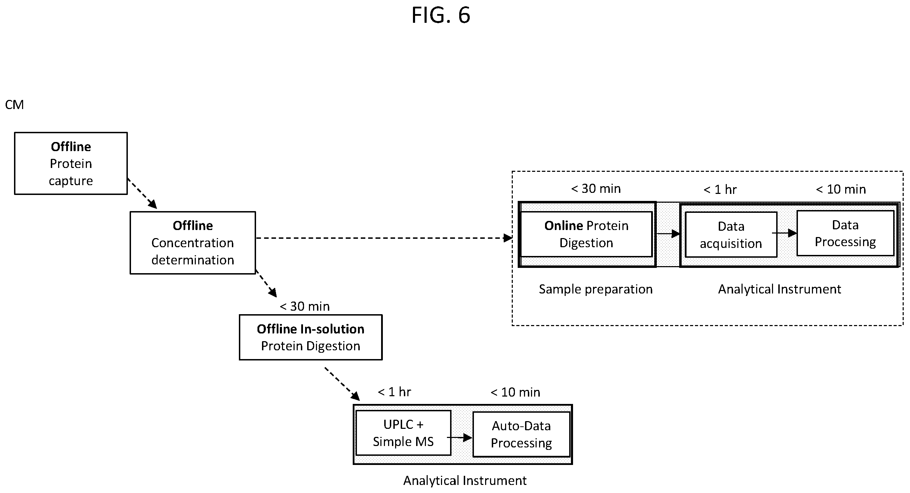

FIG. 6 is a schematic of a method described herein in which some of the steps are performed manually or offline. In one aspect, the protein capture and concentration determination steps for the sample preparation are performed offline and the protein digestion, data acquisition and data processing steps are performed online. In one aspect, sample preparation steps, including, for example, protein capture, concentration determination and protein digestion steps, are performed offline and the data acquisition and data processing steps are performed online.

In one aspect, a method for detecting a post-translational modification (PTM) of a protein is provided. In one aspect, the method is carried out by a flow injection analyzer (FIA) that is controlled by at least one processor. In one aspect, the flow injection analyzer (FIA) is a sequential injection analyzer (SIA). In one aspect, the flow injection analyzer (FIA) is a direct injection analyzer (DIA). An example of a method or system for online, automated flow injection analysis is shown in FIGS 1-4, as described above.

In one aspect, the method includes receiving a first sample that includes a protein of interest in a holding reservoir of a FIA, e.g., FIA 150 as described above. In one aspect, the first sample is obtained from a bioreactor, e.g., bioreactor 140 as described above. In one aspect, the bioreactor 140 is coupled to the FIA. In one aspect, the FIA includes a sampling device that is coupled to the bioreactor 140 and configured to obtain cell culture media or clarified cell culture media from the bioreactor 140. In one aspect, an automated system interface used to couple the bioreactor 140 and FIA, for example, a Modular Automated Sampling Technology (MAST^{™}) sampling system (Lonza, Bend, OR, U.S.A.) or a Seg-Flow automated sampling system (BioProcess International Europe, Vienna, Austria). In one aspect, the sample is manually obtained from the bioreactor and introduced into the holding reservoir of the FIA, for example, by introducing the holding reservoir from the FIA into a receptacle containing the cell culture sample. In one aspect, the first sample includes cell culture media. In one aspect, the first sample includes clarified cell culture media. In one aspect, the first sample has a volume from about 20 µL to about 1000 µL.

In one aspect, the method includes a first protein capture step. In one aspect, the protein capture step includes an affinity chromatography step. In one aspect, the affinity chromatography step includes column equilibration, sample loading and sample elution. In one aspect, the column equilibration step includes delivering, via the FIA, a first volume of a binding buffer to an affinity chromatography column in the FIA. In one aspect, the sample loading step includes transferring, via the FIA, the first sample from the holding reservoir to the affinity column. In one aspect, the sample elution step includes delivering, via the FIA a first volume of an elution buffer to the affinity chromatography column and eluting the protein in the first sample from the affinity chromatography column to form a first eluate. In one aspect, the protein in the first sample is eluted from the affinity chromatography column to form the first eluate in based on a predetermined elution time. In one aspect, the predetermined elution time is controlled by a process control manager of a process control device associate with the automated control system. In one aspect, the protein in the first sample is eluted from the affinity column to form the first eluate based on a predetermined elution volume. In one aspect, the elution volume is controlled by a process control manager of a process control device associated with the automated control system.

In one aspect, shown in FIG. 6, the protein capture step is performed manually. In one aspect, the protein capture step is performed offline. In one aspect, the protein capture step is performed at-line. In one aspect, the affinity chromatography step is performed manually. In one aspect, the affinity chromatography step is performed offline. In one aspect, the affinity chromatography step is performed at-line.

In one aspect the first eluate is transferred, via the FIA under control of the process control manager, to a protein concentration detection unit. In one aspect, the protein capture step, from column equilibration to elution, takes from about 15 minutes to about 1 hour. In one aspect, the protein capture step takes less than about 30 minutes.

In one aspect, the method includes determining, by the at least one processor, a concentration of the protein in the first eluate. In one aspect, determining the concentration of the protein in the first eluate is performed by the at least one processor according to a predetermined calibration curve. In one aspect, the concentration of protein in the first eluate is determined by measuring absorbance of the sample and calculating protein concentration based on a predetermined calibration curve. For example, a process control manager may cooperate with a data collection manager to obtain UV absorbance data from a spectrophotometer and may cooperate with a calibration manager to obtain and apply a predetermined calibration curve to the obtained UV absorbance data. In one aspect, the concentration of protein in the first eluate is determined by measuring UV absorbance of the sample from about 190 nm to about 280 nm, or at about 205 nm or at about 280 nm and calculating protein concentration based on a predetermined calibration curve. In one aspect, the method includes transferring the first eluate, via action of the FIA, to a waste receptacle T106 after the protein concentration is determined. In one aspect, the concentration determination step takes from about 5 minutes to about 30 minutes, or less than about 10 minutes.

In one aspect, shown in FIG. 6, the protein concentration determination is performed manually. In one aspect, the protein concentration determination is performed offline. In one aspect, the protein concentration determination is performed at-line.

After the protein concentration of the first eluate is determined, the method includes receiving a second sample that includes the protein in the holding reservoir. In one aspect, the second sample is obtained from a bioreactor. In one aspect, the second sample includes cell culture media. In one aspect, the second sample includes clarified cell culture media. In one aspect, the second sample is obtained from the same cell culture from which the first sample was obtained. In one aspect, the first and second samples are obtained from the cell culture at substantially the same time. In one aspect, the first and second samples are obtained from the cell culture within at least about 30 minutes, about 15 minutes, about 10 minutes, about 5 minutes, about 4 minutes, about 3 minutes, about 2 minutes or about 1 minute from each other. In one aspect, the second sample has a volume from about 20 µL to about 1000 µL.

In one aspect, the method includes a second protein capture step. In one aspect, the second protein capture step includes an affinity chromatography step. In one aspect, the second affinity chromatography step includes column equilibration, sample loading and sample elution. In one aspect, the column equilibration step includes delivering, via the FIA, a second volume of a binding buffer to the affinity column. In one aspect, the sample loading step includes transferring, via the FIA, the second sample from the holding reservoir to the affinity column. In one aspect, the sample elution step includes delivering, via the FIA a second volume of an elution buffer to the affinity column and eluting the protein in the second sample from the affinity column to form a second eluate. In one aspect, the protein in the second sample is eluted from the affinity column to form the second eluate based on a predetermined elution time. In one aspect, the elution time (and, optionally, other process parameters such as elution buffer volumes, etc.) is controlled by the at least one processor, e.g., via a process control manager, to substantially match the predetermined elution time of the first eluate to thereby obtain a second eluate. In one aspect, the second eluate has a protein concentration substantially similar to the protein concentration of the first eluate. In one aspect, the protein in the second sample is eluted from the affinity column to form the second eluate based on a predetermined elution volume. In one aspect, the elution time of the second sample is controlled by the process control manager to substantially match the elution time of the first sample. In one aspect, the elution volume of the second sample is controlled by the process control manager to substantially match the elution volume of the first sample.

In one aspect, shown in FIG. 6, the protein capture step is performed manually. In one aspect, the protein capture step is performed offline. In one aspect, the protein capture step is performed at-line. In one aspect, the affinity chromatography step is performed manually. In one aspect, the affinity chromatography step is performed offline. In one aspect, the affinity chromatography step is performed at-line.

In one aspect, the method includes digesting the protein in the second eluate to form a peptide mixture. In one aspect, the protein in the second eluate is enzymatically digested. In one aspect, digesting the protein in the second eluate includes introducing, via the FIA, a digestion reagent into the second eluate to form a digestion mixture. In one aspect, digesting the protein in the second eluate includes introducing, via the FIA, a digestion reagent that includes a digestion buffer, a reducing agent and a proteolytic enzyme into the second eluate to form a digestion mixture. In one aspect, the second eluate is contacted with an amount of proteolytic enzyme determined by the at least one processor, e.g., by an enzyme determination manager, based on the concentration of the first eluate. In one aspect, the method includes mixing the digestion mixture, e.g., via a syringe pump of the FIA as controlled by the process control manager. In one aspect, the method includes incubating the digestion mixture, e.g., under control of the process control manager.

In one aspect, the proteolytic enzyme includes heat-stable trypsin. In one aspect, the method includes introducing, via the FIA, a first amount of digestion buffer, reducing agent and heat-stable trypsin the second eluate and mixing to form a first digestion mixture. In one aspect, the first digestion mixture is incubated at a temperature from about 65°C to about 85°C for about 5 minutes to about 30 minutes to form an incubated mixture. In one aspect, the digestion mixture is incubated at a temperature from about 70°C to about 75°C for about 10 minutes to about 20 minutes to form an incubated mixture. In one aspect, the digestion mixture is incubated at a temperature from about 70°C to about 75°C for about 10 minutes to about 20 minutes to form an incubated mixture. Aspects of the incubation processes described above may be controlled via the process control manager.

In one aspect, the method includes introducing, via the FIA, a second amount of heat-stable trypsin to the incubated mixture and mixing to form a second digestion mixture. In one aspect, the second digestion mixture is incubated at a temperature from about 65°C to about 85°C for about 5 minutes to about 30 minutes to form a peptide mixture. In one aspect, the second digestion mixture is incubated at a temperature from about 70°C to about 75°C for about 10 minutes to about 20 minutes to form a peptide mixture. In one aspect, the second digestion mixture is incubated at a temperature from about 70°C to about 75°C for about 10 minutes to about 20 minutes to form a peptide mixture. Aspects of the incubation processes described above may be controlled via the process control manager.

In one aspect, shown in FIG. 6, the protein digestion step is performed manually. In one aspect, the protein digestion step is performed offline. In one aspect, the protein digestion step is performed at-line.

In one aspect, the method includes transferring, via the FIA, the peptide mixture to an analytical instrument, for example, a peptide mapping unit, which can include, for example, a mass analyzer and detecting a predetermined post-translational modification (PTM) of the protein.

In one aspect, an online method for monitoring post-translational modifications of a protein is provided. In one aspect, the method is carried out by a flow injection analyzer (FIA) as controlled by at a process control device including at least one processor. In one aspect, the flow injection analyzer includes at least one bi-directional pump. In one aspect, the FIA includes at least one syringe pump controlled by at least one processor. In one aspect, the FIA includes at least one multi-port valve controlled by at least one processor. In one aspect, the FIA includes two syringe pumps. In one aspect, the FIA includes two multi-port valves. In one aspect, the multi-port valve is a 10-port valve.

In one aspect, the method includes receiving a first sample that includes the protein into a holding reservoir of the FIA. In one aspect, the sample is advanced through the FIA by action of one or more syringe pump controlled by the at least one processor. In one aspect, the sample is advanced through the FIA at a flow rate from about 10 µL/sec to about 200 µL/sec.

In one aspect, a first volume of a binding buffer is introduced, via a multi-port valve, into an affinity column in the FIA by action of the multi-port valve.

In one aspect, the sample is advanced through the FIA system to an affinity column by action of a syringe pump. In one aspect, the method includes transferring, by action of a syringe pump, the first sample from the holding reservoir to the affinity column. In one aspect, the protein in the first sample binds to the affinity column. In one aspect, the method includes introducing, by action of the multi-port valve, a first volume of elution buffer into the affinity column to elute the bound protein to form a first eluate. In one aspect, the protein in the first sample is eluted from the affinity column to form the first eluate based on a predetermined elution time. In one aspect, the protein in the first sample is eluted from the affinity column to form the first eluate based on a predetermined elution volume. In some aspects, action of the syringe pump may be controlled by a process control manager.

In one aspect, the method includes transferring, by action of a syringe pump, the first eluate to a protein concentration detection unit. In one aspect, the method includes advancing, by action of a syringe pump, the first eluate to a spectrophotometer. In one aspect, the method includes determining, by the at least one processor and according to a measured UV absorbance, a concentration of the protein in the first eluate. In one aspect, the first eluate is transferred to a waste receptacle after the protein concentration is determined. In one aspect, the concentration of the protein in the first eluate is determined by the at least one processor according to a predetermined calibration curve.

In one aspect, the method includes receiving a second sample in the holding reservoir of the FIA. In one aspect, the method includes advancing the sample through the FIA by action of a syringe pump. In one aspect, the method includes delivering, via a multi-port valve, a second volume of a binding buffer to the affinity column. In one aspect, the method includes transferring, by action of a syringe pump, the second sample from the holding reservoir to the affinity column. In one aspect, the protein in the second sample binds to the affinity column. In one aspect, the method includes introducing, by action of the multi-port valve, a second volume of elution buffer into the affinity column to elute the bound protein to form a second eluate. In one aspect, the protein in the second sample is eluted from the affinity column to form the second eluate based on a predetermined elution time. In one aspect, the protein in the second sample is eluted from the affinity column to form the second eluate based on a predetermined elution volume. In one aspect, the first and second eluate have substantially the same elution volume. In some aspects, action of the syringe pump and the multi-port valve may be controlled by a process control manager.

In one aspect, the method includes advancing, by action of the syringe pump, the second eluate to a digestion chamber in the FIA. In one aspect, the method includes introducing, by action of the multi-port valve, a digestion reagent into the second eluate and digesting the protein in the second eluate to form a peptide mixture. In one aspect, digesting the protein includes introducing, by action of the multi-port valve, a digestion buffer, a reducing agent, a proteolytic enzyme, or a combination thereof into the second eluate in the digestion chamber to form a first digestion mixture. In one aspect, the method includes mixing the first digestion mixture. In one aspect, the digestion chamber is an actuator syringe, for example, an interior barrel of an actuator syringe, and the first digestion mixture is mixed by depressing and retracting a plunger of a syringe. In one aspect, the first digestion mixture is incubated in the digestion chamber to form an incubated mixture. In one aspect, the method includes introducing, by action of the multi-port valve, a second volume of proteolytic enzyme into the first incubated mixture to form a second digestion mixture. In one aspect, a second volume of digestion buffer is introduced into the first incubated mixture. In one aspect, the second digestion mixture is incubated to form a peptide mixture. In one aspect, the method includes mixing the second digestion mixture. In one aspect, the second digestion mixture is mixed by depressing and retracting a plunger of a syringe. In one aspect, an amount of proteolytic enzyme introduced in into the second digestion mixture is determined by the at least one processor based on the concentration of the first eluate. In one aspect, action of the syringe pump and the multi-port valve may be controlled by a process control manager.

In one aspect, the proteolytic enzyme includes heat-stable trypsin. In one aspect, the method includes introducing, by action of the multi-port valve, a first amount of digestion buffer, reducing agent and heat-stable trypsin into the second eluate and mixing to form a first digestion mixture. In one aspect, the method includes, incubating the first digestion mixture at a temperature of about 70°C to about 75°C for about 10 minutes to about 20 minutes. In one aspect, the method includes introducing, by action of the multi-port valve, a second amount of digestion buffer and heat-stable trypsin to the first digestion mixture and mixing to form a second digestion mixture. In one aspect, the method includes incubating the second digestion mixture at a temperature of about 70°C to about 75°C for about 10 minutes to about 20 minutes to form a peptide mixture.

In one aspect, the method includes advancing, by action of the syringe pump, the peptide mixture to a peptide mapping unit and detecting a post-translational modification (PTM) of the protein. In one aspect, the method includes advancing, by action of the syringe pump, the peptide mixture to a peptide mapping unit and detecting a predetermined PTM of the protein.

### C. Cell culture

In one aspect, the method is used to detect a post-translational modification (PTM) of a protein in a sample. In one aspect, the protein is a recombinant protein. In one aspect, the protein is a therapeutic protein. In one aspect, the protein is a recombinantly produced therapeutic protein. In one aspect, the sample is obtained from a cell culture.

Methods for expressing a recombinant protein in a cell culture are known. Generally, an expression vector is prepared that includes a coding sequence that encodes the protein. In one aspect, the coding sequence is operatively linked one or more regulatory sequences. Regulatory sequences are known and include, but are not limited to, promoters, enhancers and other expression control elements that control transcription or translation of the coding sequence. "Operatively linked" or "operably linked" means that the regulatory sequences are placed in the appropriate positions relative to the coding sequence to effect expression of the coding sequence. In one aspect, the regulatory sequence is contiguous with the coding sequence. In one aspect, the regulatory sequence acts in trans or at a distance to control expression of the coding sequence. In one aspect, the recombinant expression vector includes one or more additional sequences, for example, an origin of replication and/or a selectable marker.

In one aspect, the recombinant protein includes more than one polypeptide chain. In one aspect, the recombinant protein is an antibody. For a protein that includes more than one polypeptide chain, the coding sequences of the individual polypeptide chains can be inserted into the same or separate expression vectors. Methods for inserting genes into expression vectors are known. In one aspect, the recombinant expression vector encodes a signal peptide to facilitate secretion of the protein from a host cell.

To recombinantly express a protein, a host cell is transfected with a recombinant expression vectors encoding the protein. Transfection methods are known.

Host cells can include prokaryote, yeast, or higher eukaryote cells. In one aspect, the host cell is a mammalian host cell. Suitable mammalian host cells include, but are not limited to, Chinese Hamster Ovary (CHO cells), NS0 myeloma cells, COS cells, SP2 cells, COS-7 cells, human embryonic kidney cells (HEK 293 cells), baby hamster kidney cells (BHK), mouse sertoli cells (TM4), monkey kidney cells (CV1), African green monkey kidney cells (VERO-76), human cervical carcinoma cells (HELA), canine kidney cells (MDCK), buffalo rat liver cells (BRL), human lung cells (W138), human liver cells (Hep G2), human hepatoma cells (Hep G2), mouse mammary tumor cells (MMT), TRI cells, MRC 5 cells, and FS4 cells. In one aspect, the mammalian host cell is a CHO, HEK 293, COS, NS0, SP2 or PER.C6 host cell.

During cell culture, the transformed host cell is cultured in a suitable cell culture media under suitable culture conditions, for example, temperature, pH, agitation, and dissolved oxygen levels, for expression of the recombinant protein. Commercially available cell culture media include, but are not limited to, Ham's F10^{™} (Sigma), Minimal Essential Medium^{™} (MEM), (Sigma), RPMI-1640 (Sigma), and Dulbecco's Modified Eagle's Medium^{™} (DMEM), (Sigma), Iscove's Modified Dulbecco's Medium, and Minimal Essential Medium-alpha (MEM-alpha). In one aspect, the cell culture media is supplemented with hormones and/or other growth factors, salts, buffers, nucleotides, antibiotics, trace elements, and glucose or other energy sources.

In one aspect, the host cell is cultured in a small-scale bioreactor. In one aspect, the host cell is cultured in an individual flask, shaker flask or a wave bag. In one aspect, the host cell is cultured in a bioreactor having a volume from about 125 ml to about 500 ml. In one aspect, the host cell is cultured in a large-scale bioreactor. In one aspect, the host cell is cultured in a bioreactor having a volume from about 100 liters to about 20,000 liters, or more. In one aspect, the host cell is cultured in a stirred-stank, fixed-bed, fluidized bed, bubble column, or airlift bioreactor.

In one aspect, the cell culture is supplemented with a concentrated feed medium that contains components, such as nutrients and amino acids, during the course of the cell culture. In one aspect, the media in the bioreactor is not supplemented during the cell culture process in a batch process. In one aspect, the media in the bioreactor is supplemented with additional components, for example, nutrients or cell culture media, during the cell culture process in a fed-batch bioreactor. In one aspect, the media is continually added to the bioreactor and spent media and product are removed throughout the cell culture process in a perfusion bioreactor.

In one aspect, the method includes obtaining a first and a second sample from a cell culture. In one aspect, the first and second samples are obtained from the same cell culture. In one aspect, the first and second samples are obtained from the cell culture substantially at the same time. As used herein, two actions that are performed "substantially at the same time" can mean that the two actions are performed simultaneously, but also includes situations when the two actions are performed within a short time of each other. In one aspect, the first and samples are obtained within about 30 minutes of each other. In one aspect, the first and samples are obtained within about 15, about 10, about 5, about 4, about 3, about 2 or about 1 minute of each other.

### D. Recombinant protein

In one aspect, the method is used to detect a post-translational modification (PTM) of a protein of interest in a sample. In one aspect, the protein is recombinantly produced. In one aspect, the recombinantly produced protein includes at least about 1000 amino acid residues. In one aspect, the recombinantly produced protein has a molecular weight of at least about 10 kDa, or at least about 20 kDa or at least about 30 kDa.

In one aspect, the protein is a glycoprotein. In one aspect, the protein is a therapeutic antibody or antigen binding fragment thereof. In one aspect, the protein is a receptor or a receptor ligand. In one aspect, the protein is a fusion protein.

The recombinant protein can be produced intracellularly, in the periplasmic space, or directly secreted into the cell culture medium. When the polypeptide protein is produced intracellularly, the first step of a purification process typically involves lysis of the cell, for example, by mechanical shearing, osmotic shock, or enzymatic treatment, which results in the release of the entire contents of the cell into the homogenate and produces cellular debris that can be removed by differential centrifugation or by filtration.

In one aspect, the protein is a therapeutic protein. As used herein, a "therapeutic protein" is a protein that has one or more biological activity that is useful for treating, preventing or ameliorating a disease or at least one symptom of a disease.

### E. Post-translational modifications

"Post-translational modification" (PTM) refers to one or more covalent modifications made to a polypeptide during or after protein synthesis. In one aspect, the PTM is introduced by an enzyme or enzyme pathway. PTM can result in varying levels of protein heterogeneity which can significantly impact protein structure and function, including, but not limited to, biological activity, binding activity, pharmacokinetics (PK), pharmacodynamics (PD) and immunogenicity of the recombinantly produce protein. Some PTM may be beneficial to the physicochemical properties of the therapeutic protein, while others may be detrimental. The quantity and type of PTM can be influenced by cell culture conditions and are considered a critical quality attribute (CQA) that must be monitored during the manufacture of recombinantly produced therapeutic proteins.

In one aspect, a method is provided for detecting a PTM of a protein. In one aspect, a method is provided for detecting a PTM of a recombinantly produced protein. In one aspect, a method is provided for detecting a PTM of a therapeutic protein.

In one aspect, the PTM includes glycosylation. In one aspect, glycosylation includes N-linked glycosylation, O-linked glycosylation or a combination thereof. In one aspect, the PTM includes oxidation, deamidation, isomerization, glycation or a combination thereof. In one aspect, the PTM includes C-terminal modifications, including, but not limited to, lysine retention, glycine loss, amide formation or a combination thereof. In one aspect, the PTM includes N-terminal modifications, including, but not limited to, leader sequence retention, N-terminal cyclization or a combination thereof.

In one aspect, the PTM includes an N-linked glycan. In one aspect, the PTM includes an O-linked glycan. In one aspect, the PTM includes an agalactosyl glycans with 0 terminal galactose moieties, for example, G0, G0-GlcNAc, G0F, G0F-GlcNAc, or a combination thereof. In one aspect, the PTM includes a glycan with 1 terminal galactose moiety, for example, G1, G1F, G1F-GlcNAc, or a combination thereof. In one aspect, the PTM includes a glycan with 2 terminal galactose moieties, for example, G2F, G2F +Sialic Acid, or a combination thereof.

In one aspect the PTM includes a high mannose glycan. As used herein, "high-mannose glycan" refers to an N-glycan structure containing about 3, about 4, about 5, about 6, about 7, about 8, or about 9 mannose residues, which can be referred to as Man4, Man5, Man6, Man7, Man8, or Man9 glycans, respectively.

### F. Sample Preparation

Provided herein is an automated online system or method for detecting a post-translational modification (PTM) in a polypeptide. In one aspect, a method or system is provided for detecting PTM in a recombinantly produced polypeptide. In one aspect, a method or system is provided for detecting PTM in a protein. In one aspect, a method or system is provided for detecting PTM in a recombinantly produced protein. In one aspect, a method or system is provided for detecting PTM in a therapeutic protein. In one aspect, a method or system is provided for detecting PTM in a recombinantly produced therapeutic protein.

In one aspect, an automated online sample preparation system or method is provided. In one aspect, the automated online sample preparation system or method includes one or more of a protein capture step, a concentration determination step, and a protein digestion step. In one aspect, an automated, online sample preparation system or method is provided in which a protein in a sample is prepared for an analytical process, for example, mass spectrometry. In one aspect, an automated, online sample preparation system or method is provided in which a protein in a sample is prepared for an analytical process, for example, to detect one or more PTM. In one aspect, the automated, online sample preparation system or method prepares a protein in a sample for mass spectrometry (MS). In one aspect, the automated, online sample preparation system or method prepares a protein in a sample for liquid chromatography/mass spectrometry (LC/MS). In one aspect, the automated, online sample preparation system or method prepares a protein in a sample for an automated LC/MS system. In one aspect, the automated, online sample preparation system or method prepares a protein in a sample for an integrated LC/MS system.

A schematic overview of the online, automated method is provided in FIG. 5. Briefly, clarified cell culture media is delivered to a sample preparation unit that includes a protein capture unit, a protein concentration determination unit, and a protein digestion unit. The prepared sample is then transferred to an analytical instrument, for example, a peptide mapping unit that includes a data acquisition unit and a data processing unit. In one aspect, the data acquisition unit includes liquid chromatography (LC) and mass spectrometry (MS).

As shown in FIG. 6, some of the method steps can be performed manually, or offline. In one aspect, the protein capture and concentration determination steps for the sample preparation are performed offline and the protein digestion, data acquisition and data processing steps are performed online. In one aspect, sample preparation steps, including, for example, protein capture, concentration determination and protein digestion steps, are performed offline and data acquisition and data processing steps are performed online.

### 1. Flow injection analysis

In one aspect, the automated sample preparation is performed using a flow injection analyzer (FIA), optionally included within an automated process control system including one or more process control devices, as discussed with respect to FIGS. 1 and 2. Flow injection analyzers are commercially available, for example, from FIAlab Instruments, Inc. (Seattle, WA, U.S.A.). In one aspect, the FIA is a sequential injection analyzer (SIA). In one aspect, the FIA is a direct injection analyzer (DIA). In one aspect, the FIA includes a bi-directional pump and a valve. In one aspect, the FIA includes more than one pump and more than one valve. In one aspect, the FIA includes two pumps and two valves. In one aspect, the bi-directional pump is a syringe pump. In one aspect, the valve is a rotary valve. In one aspect, the valve is a multi-port valve. In one aspect, the valve is a 10-port rotary valve. In one aspect, the flow injection analyzer is a ProSIA dual pump injection analyzer (FIAlab Instruments, Inc., Seattle, WA, U.S.A.)

The following discussion of the automated process as implemented by the automated process control system 100, including at least one process control device 200, at least one bioreactor 140, at least one FIA 150, and at least one analytical instrument 160, may be understood with reference to FIGS. 1-4 and Table 1. The arrangement of reservoirs, inputs, outputs, sinks, drains, and equipment with respect to the various input and output ports of the first valve 301 and the second valve 401 of the FIA 150 is by way of example only. Other arrangements and organizations may be employed without departing from the method and system described herein. For example, the roles of the first valve 301 and the second valve 401, the first syringe pump 302 and the second syringe pump 402 may be swapped. In another example, alternate or additional ports and/or connections may be used. In the following discussion, it is understood that the first valve 301, the second valve 401, the first syringe pump 302, the second syringe pump 402, and any other automated aspects of the system are controlled by the process control device 200, as discussed above with respect to FIG. 2.

In one aspect, a sample is introduced into a holding reservoir of the FIA. The sample may be introduced by any appropriate means, including manual and automated methods. In one aspect, the bioreactor 140 is coupled to the FIA. In one aspect, the FIA includes a sampling device that is coupled to the bioreactor 140 and configured to obtain cell culture media or clarified cell culture media from the bioreactor 140. In one aspect, an automated system interface used to couple the bioreactor 140 and FIA, for example, a Modular Automated Sampling Technology (MAST^{™}) sampling system (Lonza, Bend, OR, U.S.A.) or a Seg-Flow automated sampling system (BioProcess International Europe, Vienna, Austria). In one aspect, the sample is manually obtained from the bioreactor and introduced into the holding reservoir of the FIA, for example, by introducing the holding reservoir from the FIA into a receptable containing the cell culture sample. In one aspect, a sample having a volume from about 20 µL to about 1000 µL is introduced into a holding reservoir of the FIA. In one aspect, the sample is obtained from a cell culture. In one aspect, the sample includes cell culture media. In one aspect, the sample includes clarified cell culture media. In one aspect, the cell culture media is clarified by centrifugation or filtration. In one aspect, filtration includes microfiltration or depth filtration. In one aspect, the sample includes a polypeptide. In one aspect, the host cells in the cell culture are removed from the bioreactor. In one aspect, the host cells in the cell culture are removed during cell culture, for example, in a perfusion bioreactor. In one aspect, the sample includes a recombinantly produced polypeptide. In one aspect, the sample includes a protein. In one aspect, the sample includes a recombinantly produced protein. In one aspect, the sample includes a recombinantly produced therapeutic protein.

### 2. Protein capture

In one aspect, the method includes a protein capture step in which a protein of interest in the sample is purified, for example, to separate the protein of interest from impurities such as host cell proteins or other product-related impurities present in the sample. In one aspect, the sample includes clarified cell culture media from which cells and other cellular debris have been removed. In one aspect, the protein capture step includes affinity chromatography.

"Affinity chromatography" refers to chromatographic process in which a biomolecule of interest, for example, a protein, in a mobile phase is retained by the stationary phase of the chromatographic column based on a specific, reversible binding interaction between the component of interest and a compound immobilized on the chromatographic matrix. In one aspect, affinity chromatography includes a column equilibration step, a sample loading step and a sample elution step. In one aspect, the chromatography column has a volume from about 50 µL to about 300 µL, or from about 50 µL to about 200 µL. In one aspect, affinity chromatography includes low pressure affinity chromatography. In one aspect, affinity chromatography is run at a flow rate from about 10 uL/sec to about 100 uL /sec.

In one aspect, the method includes a column equilibration step, a loading step, a wash step and an elution step. In one aspect, the same buffer is used in one or more of the column equilibration, loading and wash steps. In one aspect, a different buffer is used in one or more of the equilibration, loading and wash steps. In one aspect, the term "binding buffer" is used to refer to a buffer that is used as an equilibration buffer, a loading buffer and/or a wash buffer.

In one aspect, the protein capture step takes from about 15 minutes to about 2 hours. In one aspect, the protein capture step takes from about 15 minutes to about 1 hour. In one aspect, the protein capture step takes less than about 30 minutes.

In one aspect, the column is equilibrated by passing an equilibration buffer over the column before the sample is loaded. In one aspect, the equilibration buffer can be referred to as a binding buffer. In one aspect, the column is equilibrated with about 5 to about 10 column volumes (CVs) of equilibration buffer. In one aspect, the column is equilibrated with an equilibration buffer at flow rate from about 10 µL/sec to about 200 µL/sec. Binding buffer (or equilibration buffer) may be accessed by the FIA 150 via the S201 input port and routed through the FIA 150 to the affinity column T105.

In one aspect, the sample is loaded onto the equilibrated column. In one aspect, the sample is loaded in a loading buffer having the same composition as the equilibration buffer. In one aspect, the term "binding buffer" refers to a buffer that is used as both an equilibration buffer and a loading buffer. In one aspect, from about 20 µL to about 1000 µL of sample is loaded onto the column in a loading buffer. In one aspect, the sample is loaded onto the affinity column at flow rate from about 10 µL/sec to about 200 µL/sec. The sample may be accessed by the FIA 105 at the V209 input port 304 and routed, by the first syringe pump 302, to the affinity column 313 via theV208 input port 304.

In one aspect, the method includes a column washing step, for example, to remove proteins that interact weakly or nonspecifically with the affinity chromatography resin. In one aspect, the wash buffer has the same composition as the equilibration buffer and/or the loading buffer. In one aspect, the term "binding buffer" refers to a buffer that is used as an equilibration buffer, a loading buffer and a wash buffer. In one aspect, the wash buffer has a different composition than the equilibration buffer and/or the loading buffer. In one aspect, the loaded column is washed with about 5 to about 10 column volumes (CVs) of a wash buffer. In one aspect, the loaded column is washed with a wash buffer at flow rate from about 10 µL/sec to about 200 µL/sec. In one aspect, the wash buffer is accessed by the FIA 105 at the S202 syringe pump port 305 and routed, by the second syringe pump 402, to the affinity column 313 via the S108 syringe pump port 405.

In one aspect, the protein of interest is eluted from the column in an elution step. In one aspect, the protein of interest is eluted from the column by passing an elution buffer over the column on which the sample is immobilized. In one aspect, the protein of interest is eluted from the column using an elution buffer with a different pH or salt concentration than the loading buffer. In one aspect, the elution is a gradient elution, for example, in which the composition of the elution buffer changes linearly over time. In one aspect, the elution is a step elution, for example, in which the composition of the elution buffer is constant at each step. In one aspect, the elution time for the sample is predetermined. As used herein, "elution time" refers to the amount of time elapsed between the time when the sample is loaded onto the column and the time at which the sample is eluted from the column. In one aspect, the elution time is the amount of time elapsed between the time the sample is loaded onto the column and the maximum eluate concentration. In one aspect, the elution buffer is applied to the column at flow rate from about 10 µL/sec to about 200 µL/sec to elute the protein of interest from the column. In one aspect, the solution eluted from the column that contains the protein of interest is referred to as an eluate. The elution buffer may be accessed by the FIA 105 at the V203 input port 305 and routed, by the first syringe pump 302, to the affinity column 313 via theV208 input port 304.

In one aspect, the protein is an antibody, antibody fragment, or other protein that includes an Fc region, for example, an Fc fusion protein. In one aspect, the affinity column includes an immunoglobulin-binding protein. In one aspect, the affinity column includes an immunoglobulin-binding bacterial protein. In one aspect, the affinity column includes an immunoglobulin-binding bacterial protein such as Protein A, Protein G, Protein A/G or Protein L. In one aspect, the affinity column is a Protein A column. In one aspect, the affinity column is a Protein G column. In one aspect, the affinity column is a Protein A/G column. In one aspect, the affinity column is a Protein L column. In one aspect, the column is a Protein M column.

"Protein A chromatography" refers to chromatography that involves a specific, reversible interaction between the Fc portion of an immunoglobulin molecule and an IgG binding domain of Protein A. In one aspect, protein A chromatography includes columns with protein obtained from *Staphylococcus aureas.* In one aspect, protein A chromatography includes columns with proteins produced by recombinant or synthetic methods. In one aspect, protein A chromatography includes columns with protein A variants that retain the ability to bind to the Fc region of an immunoglobulin.

In one aspect, the protein of interest includes an N- or C- terminal tag to facilitate protein purification, including, but not limited to, tags that includes streptavidin, biotin, poly-histidine (His) or glutathione-S-transferase (GST).

In one aspect, the affinity column matrix includes a monoclonal antibody that specifically binds to the recombinantly produced protein.

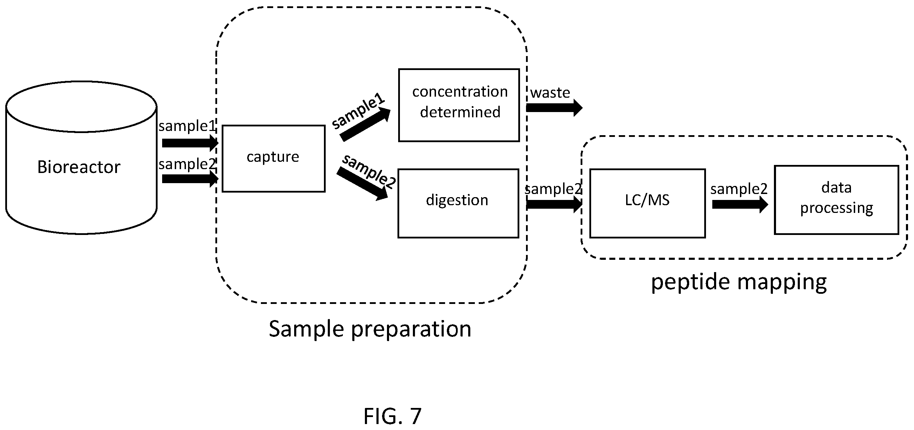

As shown in FIG. 7, the eluate obtained from the protein capture step can be transferred, via action of the FIA 150, to a protein concentration detection unit or a protein digestion step. In one aspect, a first sample is purified by affinity chromatography and the first eluate is transferred to a protein concentration determination unit. The first eluate may be drawn off the affinity column 313 by the second syringe pump 402 and transferred to the protein concentration determination unit (e.g., spectrophotometer 414) via the multi-way junction 412. In one aspect, a second sample is purified by affinity chromatography using the affinity column 313 to obtain a second eluate. The second eluate is transferred to a protein digestion step. The second eluate may be drawn off the affinity column 313 by the second syringe pump 402 and transferred to a S105 digestion chamber 415 via the V104 input port 404 of the second valve 401.

### 3. Concentration determination

In one aspect, eluate from the affinity chromatography step is transferred to a protein detection unit. In one aspect, the protein detection unit measures the concentration of a sample protein using a spectrophotometer 414. In one aspect, transfer of the eluate from the affinity column 313 to the spectrophotometer 414 may be enabled through action of the second syringe pump 402 drawing the eluate from the affinity column 313 through the multiway valve 412 via the S108 syringe pump port 405 and pushing the eluate back out through the multiway valve 412 to the spectrophotometer 414. In one aspect, the concentration of a protein in a sample is determined by measuring UV absorbance. In one aspect, the concentration of a protein in a sample is determined by measuring UV absorbance from about 190 nm to about 280 nm. In one aspect, the concentration of a protein in a sample is determined measuring UV absorbance due to the presence of the amino acids tyrosine and tryptophan. In one aspect, the concentration of a protein in a sample is determined measuring UV absorbance from about 175 nm to about 280 nm. In one aspect, the concentration of a protein in a sample is determined by measuring UV absorbance at about 280 nm. In one aspect, the concentration of a protein in a sample is determined by measuring UV absorbance due to the peptide backbone. In one aspect, the concentration of a protein in a sample is determined by measuring UV absorbance from about 190 nm to about 220 nm. In one aspect, the concentration of a protein in a sample is determined by measuring UV absorbance at about 205 nm. In one aspect, the concentration of a protein in a sample is determined by a colorimetry assay, for example, using the Bradford or Lowry method.

In one aspect, the UV absorbance of a first eluate is determined by spectrophotometry and the protein concentration is calculated based on a predetermined calibration curve. In one aspect, the concentration determination step takes from about 5 minutes to about 30 minutes, or less than about 10 minutes. UV absorbance data may be measured by the data collection manager 254 and, in conjunction with the process control manager 252 and calibration manager 256, the protein concentration may be determined as discussed above.

### 4. Automated in-solution protein digestion

In one aspect, eluate obtained from the protein capture step is transferred to a digestion chamber. In one aspect, the second eluate obtained from the protein capture step is transferred to a digestion chamber. In one aspect, the second eluate obtained from the affinity chromatography step is transferred to a digestion chamber. The second eluate may be drawn off the affinity column 313 by the second syringe pump 402 and transferred to a digestion chamber 415 via the T101 multiway valve 412, S108 syringe pump port to the S105 digestion chamber 415.

In one aspect, the protein in the eluate obtained from the affinity chromatography column is digested to form a peptide mixture for use in an analytical process, for example, peptide mapping. In one aspect, the protein in the eluate obtained from the affinity chromatography column is digested into peptides with an average size of less than about 3000 Da, less than about 2500 Da, less than about 2000 Da, or less than about 1500 Da. In one aspect, the protein in the eluate from the affinity chromatography column is digested into peptides with an average size between about 500 Da and about 5000 Da. In one aspect, the protein in the eluate from the affinity column is digested into peptides with an average size from about 1000 Da to about 5000 Da. In one aspect, the protein in the eluate from the affinity chromatography column is digested into peptides with an average size from about 500 Da, about 750 Da or about 1000 Da and up to about 1500 Da, 2000 Da, 2500 Da or about 3000 Da. In one aspect, the protein in the eluate obtained from the affinity chromatography column is digested to form a peptide mixture that includes peptides with a length from about 2 to about 100 amino acids, at least about 5 or about 10 amino acids and up to about 15, about 25, about 50 or about 100 amino acids, or about 5 to about 50, or about 10 to about 25 or about 10 to about 15 amino acids.

A conventional enzymatic protein digestion method typically includes a denaturing step, a reducing step, an alkylating step, a buffer exchange and a digesting step. Denaturing can be accomplished by increased temperature or using chemical denaturants, for example chaotropic agents, salts or detergents, including, but not limited to, guanidine hydrochloride and urea. Once the protein is denatured, disulfide bonds are reduced by adding a reducing agent, for example, dithiothreitol (DTT), glutathione, b-mercaptoethanol (b-ME), or tris(2-carboxyethyl)phosphine (TCEP). An alkylating agent, for example, iodoacetic acid, iodoacetamide, acrylamide, or chloroacetamide, is added to prevent re-formation of the disulfide bonds.

Because the reagents used in a conventional protein digestion method, for example, chemical denaturants or alkylating agents, can degrade a proteolytic enzyme, the reagents are typically removed, for example, using a buffer exchange, before addition of the proteolytic enzyme. Once the chemical denaturants and alkylating agents are removed, the proteolytic enzyme can be added to digest the protein. A conventional protein digestion process is typically performed at a neutral pH at 37°C and takes from about 12 to about 24 hours, generally around 18 hours.

Conventionally, proteolytic digestion is stopped by the addition of an acid or other chemical agent, for example, formic acid (FA), hydrochloric acid (HCl) or acetic acid (HAc); or L cyanogen bromide (CNBr), 2-nitro-5-thiocyanobenzoate (NTCB) or hydroxylamine.

Provided herein is a time-efficient streamlined protein digestion method. In one aspect, the protein digestion method is automated and online. Whereas conventional protein digestion processes can take up to 4 hours, in one aspect, the automated, online protein digestion method takes from about 5 minutes up to about 1 hour. In one aspect, protein digestion takes from about 5 minutes to about 30 minutes. In one aspect, protein digestion takes from about 15 minutes to about 30 minutes. In one aspect, protein digestion takes less than about 30 minutes. In one aspect, the online protein digestion method does not include an alkylation or buffer exchange step.

In one aspect, protein denaturation, reduction and digestion are performed in the same reaction mixture. In one aspect, protein denaturation, reduction and digestion are performed in the same digestion chamber. In one aspect, protein denaturation, reduction and digestion are performed concurrently. In one aspect, a reducing agent is introduced into the digestion chamber 415 via S103 syringe pump port. In one aspect, from about 5 µL to about 20 µL reducing agent is introduced into the digestion chamber 415 via S103 syringe pump port. In one aspect, a proteolytic enzyme, for example, trypsin, is introduced into the digestion chamber 415 via S102 syringe pump port. In one aspect, from about 5µL to about 20µL proteolytic enzyme is introduced into the digestion chamber 415 via S102 syringe pump port. In one aspect, from about 5µL to about 20µL trypsin is introduced into the digestion chamber 415 via S102 syringe pump port. In one aspect, digestion buffer is introduced into the digestion chamber 415 via the V104 input port 404 of the second valve 401. In one aspect, digestion buffer is introduced into the digestion chamber 415 via the V111 valve output port 408, S109 syringe pump port and S105 syringe pump port. In one aspect, from about 100 µL to about 400 µL digestion buffer is introduced into the digestion chamber 415. In one aspect, the reagents are combined with the second eluate in the digestion chamber using 402 Syringe 406. In one aspect, the protein in the second eluate is denatured in the digestion chamber 415.

In one aspect, protein digestion is performed at an elevated temperature. In one aspect, the protein is denatured using a high temperature denaturation. In one aspect, protein digestion is performed using a temperature resistant proteolytic enzyme. In one aspect, protein digestion is performed at an elevated temperature, for example, at a temperature from about 35°C to about 85°C. In one aspect, protein digestion is performed at a temperature from at least about 35°C, about 40°C, about 45°C, about 50°C, about 60°C, or about 70°C and up to about 75°C, about 80°C, about or about 85°C. In one aspect, protein digestion is performed at a temperature from at least about 35°C, about 36°C, about 37°C, about 38°C, about 39°C, or about 40°C and up to about 71°C, about 72°C, about 73°C, about 74°C, or about 75°C. In one aspect, protein digestion is performed at a temperature from about 50°C to about 85°C. In one aspect, protein digestion is performed at a temperature from about 65°C to about 80°C. In one aspect, protein digestion is performed at a temperature from about 70°C to about 75°C. In one aspect, the protein digestion is performed at a temperature from about 70°C to about 72°C.

In one aspect, protein digestion includes contacting a protein solution with a digestion reagent to form a digestion mixture. In one aspect, the digestion reagent includes a digestion buffer, a reducing agent, and a proteolytic enzyme. In one aspect, an eluate from an affinity column is contacted with a digestion reagent to form a digestion mixture. In one aspect, an eluate from an affinity column is contacted with a digestion reagent that includes a digestion buffer, a reducing agent, and a proteolytic enzyme to form a digestion mixture. In one aspect, the second eluate from the affinity column is contacted with a digestion buffer, a reducing agent, and a proteolytic enzyme to form a digestion mixture.

In one aspect, the digestion buffer has a pH from about 6.0 to about 8.0, or about 6.5 to about 7.5. In one aspect, the digestion buffer has a pH from about 6.5. In one aspect, the digestion buffer is from the Thermo Fisher SMART digestion kit (Thermo Fisher, Waltham, MA, U.S.A.).

In one aspect, the reducing agent includes Tris(2-carboxyethyl)phosphine (TCEP).

In one aspect, the proteolytic enzyme includes, for example, trypsin, chymotrypsin, pepsin, thermolysin, papain, pronase, endopeptidase Arg-C, peptidyl-Asp metallo endopeptidase (endopeptidase Asp-N), Glutamyl endopeptidase (Glu-C endopeptidase), and Lysyl endopeptidase (Lys-C endopeptidase). In one aspect, the proteolytic enzyme includes trypsin. In one aspect, the proteolytic enzyme includes a heat-stable trypsin. In one aspect, the proteolytic enzyme includes SMART Trypsin (Thermo Fisher Scientific, Waltham, MA, U.S.A.).

In one aspect, the reagents used in protein digestion do not interfere with mass spectrometry (MS). For example, ionization in MS can be disturbed by the presence of contaminants in the sample, including, but not limited to salt and/or detergent, particularly high concentrations of salts and/or detergents. In one aspect, the reagents used in protein digestion do not interfere with the ionization process in MS. In one aspect, the protein digestion method does not include a buffer exchange step.

Alternately, the protein digestion method includes a buffer exchange step. In one aspect, the protein digestion method includes one or more reagents that may interfere with ionization in MS, including, but not limited to: a chemical denaturing reagent, for example, a chaotropic agents such as guanidine hydrochloride and urea; a reducing agent, such as dithiothreitol (DTT) glutathione, or b-mercaptoethanol (b-ME); or an alkylating agent, such as, iodoacetic acid, iodoacetamide, acrylamide, or chloroacetamide, that are removed using a buffer exchange step. In one aspect, the buffer exchange reagent is delivered to the digestion chamber via a multi-port valve. In one aspect, the digestion method is performed at room temperature, although the process will take longer at room temperature.

In one aspect, the amount of proteolytic enzyme used in the digestion mixture for the second eluate is determined based on the protein concentration of the first eluate. In one aspect, the second eluate is contacted with trypsin at a ratio of trypsin:protein from about 1:10 to about 1:100. In one aspect, the second eluate is contacted with trypsin at a ratio of trypsin:protein from about 1:20 to about 1:30. In one aspect, the ratio of trypsin:protein is at least about 1:10 and up to about 1:100, or at least about 1:10 or 1:20 and up to about 1:30, about 1:40, about 1:50 or about 1:100, or from about 1:10 to about 1:100, about 1:10 to about 1:50, about 1:10 to about 1:40, about 1:10 to about 1:30, about 1:10 to about 1:20, about 1:20 to about 1:100, about 1:20 to about 1:50, about 1:20 to about 1:40, or about 1:20 to about 1:30.

In one aspect, the second eluate is contacted with the digestion buffer, the reducing agent and the proteolytic enzyme concurrently. As used herein, "concurrently" when used in connection with method steps described herein, refers to a method in which the steps are performed substantially at the same time, i.e., in which the execution of at least a portion of one method step overlaps in time with the execution of a portion of another method step. "Concurrently" does not require exact simultaneous activity, i.e., it is not required that all method steps begin or end at the same time. In one aspect, "concurrently" can mean that all reagents necessary for the method steps are combined in the same reaction mixture such that the reactions occur in the same reaction volume and during the same incubation period. In one aspect, the digestion mixture includes the digestion buffer, the reducing agent, the proteolytic enzyme and the second eluate. In one aspect, the digestion mixture is prepared by introducing the second eluate and the other reagents into a chamber of a syringe and the digestion mixture is mixed by depressing and retracting the plunger of the syringe. In one aspect, the digestion chamber is used as an incubation chamber. For example, the second eluate may be transferred into a chamber of the actuation syringe 406 from the affinity column 313, as discussed above. The actuation syringe 406 may further access a proteolytic enzyme via the S102 syringe pump port 405. The second eluate and the proteolytic enzyme, and any other suitable reagents, may then be mixed in a chamber of the actuation syringe 406.

In one aspect, the second eluate is contacted with the digestion buffer, the reducing agent and the proteolytic enzyme sequentially. As used herein, "sequentially" when used in connection with method steps described herein, refers to a method in which the steps are performed at different points in time, for example, in which separate events occur in the practice of the method. In one aspect, the sequential steps are performed during separate incubation periods. In one aspect, the sequential steps are performed in different reaction mixtures. In one aspect, the sequential method steps are performed at a different time, but in the same rection chamber.

In one aspect, the temperature of the incubation chamber can be elevated, for example, to incubate the digestion mixture at a temperature from about 35°C to about 85°C. In one aspect, the digestion mixture is incubated at a temperature from at least about 35°C, about 40°C, about 45°C, about 50°C, about 60°C, or about 70°C and up to about 75°C, about 80°C, about or about 85°C. In one aspect, the digestion mixture is incubated at a temperature from at least about 35°C, about 36°C, about 37°C, about 38°C, about 39°C, or about 40°C and up to about 71°C, about 72°C, about 73°C, about 74°C, or about 75°C. In one aspect, the digestion mixture is incubated at a temperature from about 50°C to about 85°C. In one aspect, the digestion mixture is incubated at a temperature from about 65°C to about 80°C. In one aspect, the digestion mixture is incubated at a temperature from about 70°C to about 55°C. In one aspect, the digestion mixture is incubated at a temperature from about 70°C to about 72°C. Temperature of the digestion chamber may be controlled as a process parameter, e.g., via the process control device.

In one aspect, incubating the digestion mixture includes incubating at a temperature from about 36°C to about 85°C for about 5 minutes to about 30 minutes. In one aspect, incubating the digestion mixture includes incubating at a temperature from about 50°C to about 75°C for about 15 minutes to about 30 minutes. In one aspect, incubating the digestion mixture includes incubating at a temperature from about 70°C to about 72°C for about 15 minutes to about 30 minutes.

In one aspect, the digestion method includes two steps: In one aspect, the first digestion mixture is incubated at a temperature from about 36°C to about 85°C for about 5 minutes to about 30 minutes. In one aspect, the first digestion mixture is incubated at a temperature from about 50°C to about 75°C for about 15 minutes to about 30 minutes. In one aspect, the first digestion mixture is incubated at a temperature from about 70°C to about 72°C for about 15 minutes to about 30 minutes. In one aspect, additional proteolytic enzyme and/or digestion buffer is added to the first digestion mixture to form a second digestion mixture. In one aspect, the second digestion mixture is incubated at a temperature from about 36°C to about 85°C for about 5 minutes to about 30 minutes. In one aspect, the second digestion mixture is incubated at a temperature from about 50°C to about 75°C for about 15 minutes to about 30 minutes. In one aspect, the second digestion mixture is incubated at a temperature from about 70°C to about 72°C for about 15 minutes to about 30 minutes.

### G. Peptide Mapping Analysis

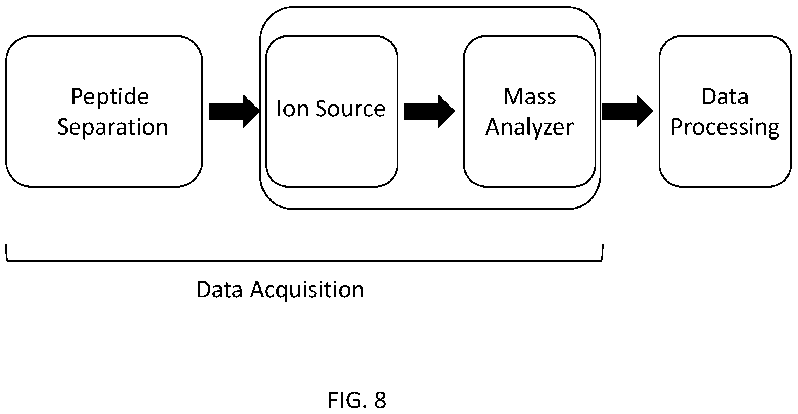

In one aspect, the peptide mixture is transferred to an analytical instrument. In one aspect, the analytical instrument includes a peptide mapping unit. In one aspect, shown schematically in FIG. 8, the peptide mapping unit includes three components: a peptide separation component, a detection or data acquisition unit; and a data processing unit. In one aspect, the data acquisition unit includes an ion source and a mass analyzer. For example, the peptide mixture may be drawn from the digestion chamber 415 by action of the second syringe pump 402 to the analytical instrument, e.g., peptide mapping unit, via the V108 input valve 404.

In one aspect, an automated peptide mapping system is used. Automated peptide mapping systems are commercially available and include, for example, the BioAccord Peptide Mapping System (Waters Corporation, Milford, MA, USA).

In one aspect, peptide mapping takes from about 30 minutes to about 2 hours, or less than 1 hour.

In one aspect, the peptide mapping unit includes a peptide separation component. In one aspect, the peptide separation component includes a chromatography column. In one aspect, the peptides in the peptide mixture are separated by column chromatography. In one aspect, column chromatography includes liquid chromatography (LC). Liquid chromatography includes, but is not limited to, ultra-performance liquid chromatography (UPLC) and high-performance liquid chromatography (HPLC). In one aspect, the peptides in the peptide mixture are separated by UPLC. In one aspect, the peptides in the peptide mixture are separated by HPLC.

In one aspect, the peptide mapping unit includes a data acquisition unit. In one aspect, the data acquisition unit includes a mass spectrometer (MS). In one aspect, the data acquisition unit includes an ion source and a mass analyzer. In one aspect, the data acquisition unit includes peptide separation instrument, an ion source and a mass analyzer. See, FIG. 8.

In one aspect, the ion source ionizes peptides in the peptide mixture. Methods for ionizing peptides are known and include, but are not limited to, electrospray ionization (ESI).

In one aspect, the mass analyzer separates the gas phase ions by mass-to-charge ratio (m/z). In one aspect, the mass analyzer includes tandem mass spectrometry (MS/MS) which includes more than one step of mass spectrometry in which fragmentation is performed between the mass spectrometry processes.

In one aspect, the peptide mapping unit includes a data processing unit. In one aspect, the data processing unit includes a processor that processes the data from the data acquisition unit to identify and/or quantify a post translational modification (PTM) of the protein. In one aspect, the peptides identified by the mass spectrometer are used as surrogate representatives of the intact protein obtained from the cell culture media. In one aspect, the data processing provides information regarding the amino acid sequence of the protein, and/or the presence, location and/or quantity of one or more PTM. In one aspect, the data processing unit provides information regarding a predetermined PTM. In one aspect, data processing takes from about 5 minutes to about 30 minutes, or less than about 10 minutes. In aspects, the data processing unit of the peptide mapping unit may interface and/or otherwise communicate with the process control device 200. In some aspects, functionality of the data processing unit of the peptide mapping unit may be carried out by software operational on the process control device 200 without need of the data processing unit.

### H. Modulation of post-translational modifications/modifications to cell culture conditions

In one aspect, a method of producing a recombinant protein is provided. In one aspect, the method includes culturing a host cell under conditions in which the recombinant protein is expressed; detecting a post-translational modification (PTM) of the protein according to a method described herein; and modifying one or more cell culture parameters based on the post-translational modification detected. In one aspect, a recombinant protein is provided that is produced by the method described herein. In one aspect, a pharmaceutical composition is provided that includes a recombinant protein produced by the method described herein, and a pharmaceutically acceptable carrier.

In one aspect, one of more of the following cell culture parameters are modified based on a PTM detected by the method described herein: pH; CO₂ level; amount of dissolved oxygen (dO₂); temperature; amount or type of nutrient; presence and types of glycan precursors, or a combination thereof. In one aspect, one or more cell culture parameters are adjusted in "real-time" based on a PTM detected by the method described herein. In one aspect, one or more cell culture parameters are adjusted in the cell culture or bioreactor from which the sample was taken. In one aspect, one or more cell culture parameters are adjusted in the cell culture or bioreactor within less than about 1 day, about 12 hours, about 6 hours, about 5 hours, about 4 hours, about 3 hours or about 2.5 hours after a sample is taken from the cell culture or bioreactor.

### I. Incorporation by Reference

All references cited herein, including patents, patent applications, papers, text books and the like, and the references cited therein, to the extent that they are not already, are hereby incorporated herein by reference in their entirety.

### WORKING EXAMPLES

### Example 1. Automated sample preparation

Recombinant cells expressing a sample IgG1 monoclonal antibody (IgG1 mAb) were cultured in a perfusion bioreactor. The cell culture media (CM) was processed in the perfusion bioreactor to remove cells and cellular debris to obtain a clarified cell culture media (CCM), which was introduced into a holding reservoir of a ProSIA Dual Pump Injection Analyzer (FIAlab^{®} Instruments, Inc., Seattle, WA, U.S.A.). Proprietary SIAsoft^{®} software (FIAlab^{®} Instruments, Inc., Seattle, WA, U.S.A.) was used to control the system components and for data collection and analysis. Sample and reagents were introduced via two syringe pumps (syringe pump 1 and syringe pump 2) and their associated 10-port valves (valve 1 and valve 2, respectively).

0.3 mL of a first sample of CCM was obtained from the holding reservoir and injected, via valve 1 of the FIA, into a Protein A affinity column that was pre-treated with equilibration buffer. IgG1 mAb from the first sample was eluted from the Protein A column as follows: 500 µL binding buffer was injected into the affinity column at 50µL/sec to 100 µL/sec. 300 µL of CM was injected at 50µL/sec to 100 µL/sec. 1 mL binding buffer was injected for wash at 50µL/sec to 100 µL/sec. 100-300 µL elution buffer was injected at 20 µL/sec.

The eluate was collected and transferred, via the FIA, to a UV spectrometer where the absorbance was measured at 280 nm to determine the concentration of the IgG1 mAb in the first sample.

300 µL of a second sample of CCM (or the same volume as the first sample of CCM) was obtained from the holding reservoir and injected, via valve 1of the FIA, into the Protein A affinity column that was pre-treated with an equilibration buffer. IgG1 mAb from the second sample was eluted from the Protein A column using a gradient elution as described above. The eluate was collected and transferred, via the FIA, to the barrel of syringe pump 2.

0.01 mL of Tris(2-carboxyehtyl)phosphine) (TCEP) (concentration 500 mM), 0.3 mL digestion buffer and trypsin were injected into the barrel of syringe pump 2, via valve 2 of the FIA. The amount of trypsin that was added was calculated based on protein concentration determined for the first sample. The digestion reagents were then mixed by depressing and retracting the plunger of the syringe and incubated the mixture for 15 minutes at 70°C to obtain a sample containing trypsin digested peptides ("tryptic peptides).

### Example 2. Online peptide mapping

Peptide mapping of the tryptic peptides from FIA sample preparation in Example 1 were analyzed by liquid chromatography coupled with UV detection and mass spectrometry using a Waters BioAccord^{™} integrated peptide mapping system that included an ACQUITY^{™} UPLC^{™} I-Class PLUS system equipped with an ultraviolet (TUV) detector coupled in-line to an ACQUITY RDa^{™} mass spectrometer.

Chromatographic conditions were as follows: temperature 65°C; mobile phase A: 0.1% formic acid in water; mobile phase B: 0.1% formic acid in acetonitrile; gradient elution (100%-40%); and flow rate: 0.2 ml/min. The run time for mobile phase A was 40 minutes. UV detection at 220nm.

For mass spectrometry, the peptides were detected using MS and tandem MS (MS/MS) detection at a mass range of 50-2000 m/z.

### Example 3. Protein Concentration Determination

The feasibility of determining protein concentration of the Protein A eluate was evaluated.

A calibration curve was generated by injecting reference standards (RS) with known concentrations onto the affinity column at a known volume. Blank, Calibrant 1(RS at 0.1 mg/mL), Calibrant 2 (RS at 0.5 mg/mL), Calibrant 3 (RS at 1.0 mg/mL), and Calibrant 4 (RS at 2.0 mg/mL) were injected separately into the affinity column. The UV response read-out was recorded in a built-in calibrant table. A calibration curve was generated with a calculated slope and coefficient. The calibration data was saved as calibration file.

Before the digestion process was performed, the calibration file was opened within the FIA software. Then, a cell culture media sample with an unknown concentration was injected into the affinity column. The UV response was obtained and entered into the calibration file to calculate the protein concentration. Live feedback of titer information was populated and adapted to enzyme calculation while the digestion method was running.

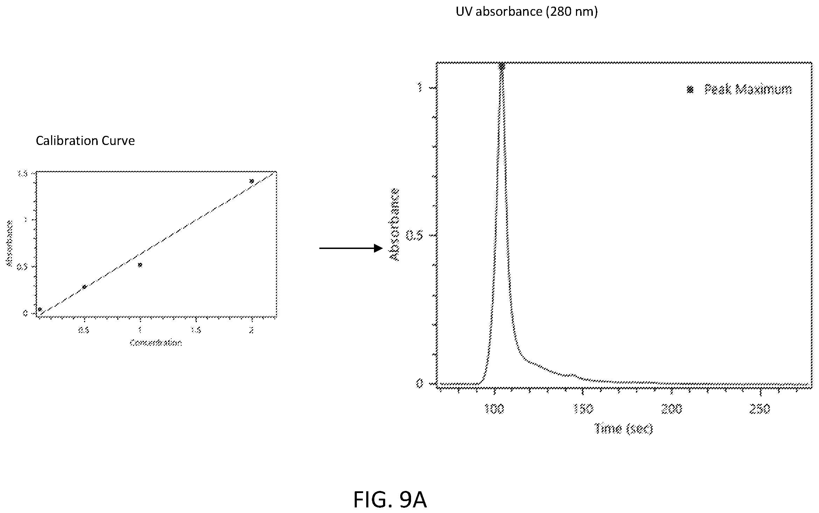

A sample calibration curve is shown in FIG. 9A, with concentrations in the x-axis and corresponding UV responses on y-axis. Each calibration point in the calibration curve on the left represents one UV measurement of known concentration of the reference standard (RS) for the IgG sample. The UV profile of the affinity column is shown in the right panel. The calibration curve was generated using 4 different concentrations of reference standard (RS). 1^{st} order of polynomial fit model was used to calculate the slope of calibration curve.

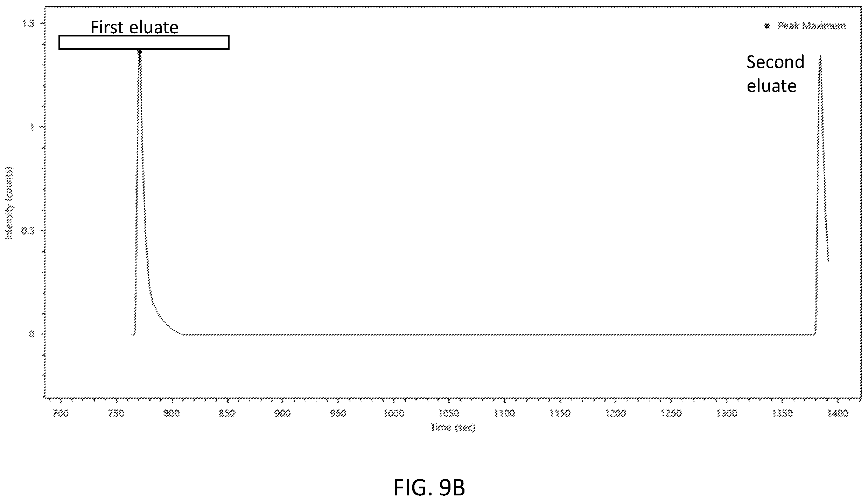

FIG. 9B is a UV profile used for making a concentration determination for a first eluate and showing that the same amount of protein was captured for the second eluate, which was then used in for protein digestion.

### Example 4. Glycan profiling: online method compared to glycan release

Glycan trending determined using the online peptide mapping method was compared to glycan trending results using a conventional glycan release method (2-aminobenzamide (Oligo 2-AB) glycan detection). Briefly, the glycosylation profile for a sample monoclonal IgG1 was determined using the online peptide mapping method described in Example 1 and a ProZyme 2AB labeling kit (Agilent, Santa Clara, CA, U.S.A.) following the manufacturer's instructions.

Briefly, a sample containing the IgG1 was treated with PNGase F enzyme to cleave the glycans from protein backbone. The released glycans were purified and collected using a solid phase extraction preparation kit. After preparing the 2AB labeling reagents, they were combined with the glycans and incubated at 65°C for 3 hours. The labeled glycans were then cleaned to remove access reagent and dried.

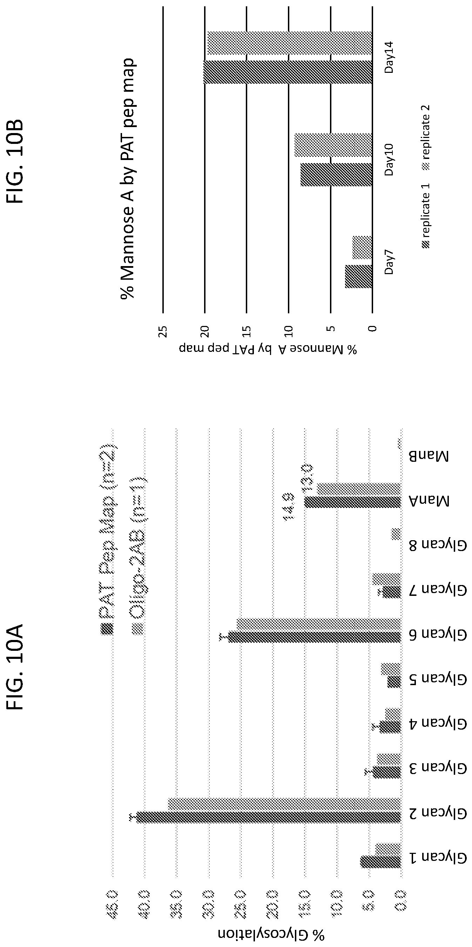

Glycosylation for 10 glycans (G0, G0F, G0F-G1cNAc, G0-G1cNAc, G1, G1F, G2F, G2f + SA, Man5 and Man6) was determined using the online peptide mapping method described in Example 1 and the Oligo 2-AB glycan release method. As shown in FIG. 10A, the glycosylation profile determined using the online method demonstrated a similar trending to the glycosylation profile determined used the Oligo-2AB procedure. FIG. 10B shows the % quantification of one of glycan species (mannose) monitored during cell culture time. Replicates of each time point show a constant level of % quantification.

### Example 5. Glycan profiling: online method compared to glycan release

Glycan trending for was determined for a sample IgG1 mAb obtained from four different bioreactors at three time points (12 time-points in all) using the online peptide mapping method and the Oligo 2-AB glycan release method following the procedures described in Example 4. The two methods have a direct linear relationship, indicating that the results were comparable for the types of glycosylation tested.

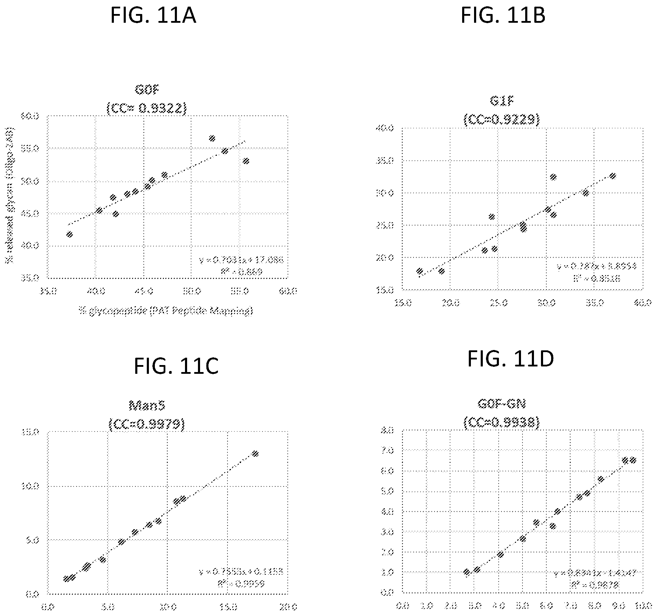

FIG. 11 A-E show the correlation between the two methods for five glycans: G0F (CC=0.9322); G1F (CC=0.9229); Man 5 (CC=0.9979); C0F-GN (CC=0.9938); and G2F+SA (CC=0.8488, within a range of assay variability and limit of quantification (LOQ)).

### Example 6. Detection of Post Translational Modification

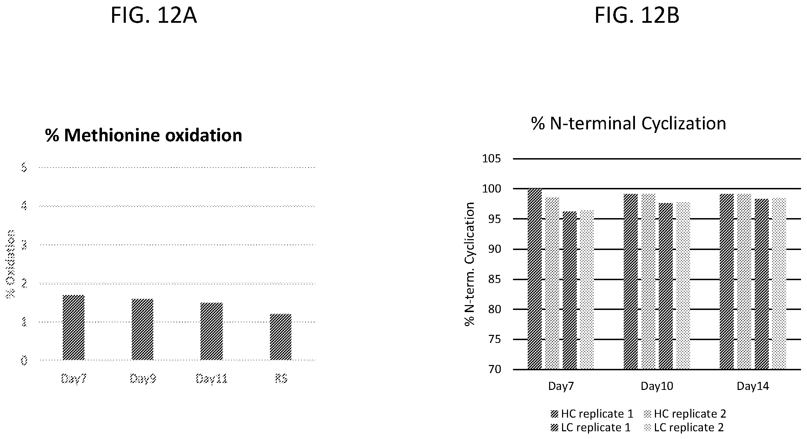

Oxidation is common post translational modification found in various methionine sites within monoclonal antibodies. Methionine oxidation is an important product quality attribute that is monitored during cell culture processes. FIG. 12A shows that the level of oxidation at a particular methionine residue was maintained below 2% when measured at Day 7, 9 and 11 of cell culture as compared to a reference standard (RS).

N-terminal cyclization is a common post translation modification found in the N-terminal sequence of antibody heavy and light chains. FIG. 12B shows that level of N-terminal cyclization remained above 95 % when measured at Day 7, 10 and 14 of cell culture. Replicates of each time point shows the constant level of modifications in heavy and light chains.

### Example 7. Site-specific glycosylation

In contrast to the conventional Oligo 2-AB glycan release method described in Example 2, the online peptide mapping method described in Example 1 can be used to obtain site-specific glycosylation information.

In a situation in which a molecule has more than one glycosylation site, peptide mapping analysis can provide site-specific glycosylation information. For example, the amino acid where the glycosylation occurred can be determined at the peptide level to provide information about the site-specific glycosylation of the intact protein. In contrast, in the glycan release method, all site-specific information about glycosylation is lost when the glycans are cleaved from the protein for analysis.

## Claims

1. A method for detecting a post-translational modification of a protein which is a recombinantly produced antibody, antibody fragment, or Fc fusion protein, the method to be carried out by a flow injection analyzer (FIA) controlled by at least one processor, the method comprising:
(a) receiving a first sample comprising the protein in a holding reservoir;
(b) delivering, via the FIA, a first volume of a binding buffer to a protein A affinity column in the FIA;
(c) transferring, via the FIA, the first sample from the holding reservoir to the protein A affinity column;
(d) delivering, via the FIA, a first volume of an elution buffer to the affinity column thereby eluting the protein in the first sample from the affinity column to form a first eluate;
(e) transferring, via the FIA, the first eluate to a protein concentration detection unit;
(f) determining, by the at least one processor, a concentration of the protein in the first eluate according to a predetermined calibration curve;
(g) receiving a second sample comprising the protein in the holding reservoir;
(h) delivering, via the FIA, a second volume of the binding buffer to the affinity column;
(i) transferring, via the FIA, the second sample from the holding reservoir to the affinity column;
(j) delivering, via the FIA, a second volume of an elution buffer to the affinity column thereby eluting the protein in the second sample from the affinity column to form a second eluate;
(k) transferring, via the FIA, the second eluate to a digestion chamber;
(l) delivering, via the FIA, a digestion reagent to digest the protein in the second eluate to form a peptide mixture; and
(m) transferring, via the FIA, the peptide mixture to a peptide mapping unit and detecting the post-translational modification of the protein;
wherein the first and second samples are obtained from a cell culture in a bioreactor, and optionally wherein the bioreactor is a batch, fed-batch or perfusion bioreactor.

2. The method of claim 1, wherein the FIA is a sequential injection analyzer (SIA) or a direct injection analyzer (DIA), wherein the protein concentration detection unit optionally comprises a spectrophotometer, and, optionally, wherein the spectrophotometer measures UV absorbance of the first eluate and, by the at least one processor, calculates a protein concentration based on a calibration curve.

3. The method of claim 1, further comprising controlling an elution time of the protein in the first sample eluted from the affinity column to form the first eluate in (d) by the at least one processor based on a predetermined elution time.

4. The method of claim 3, further comprising controlling an elution time of the protein in the second sample eluted from the affinity column to form the second eluate in (j) by the at least one processor based on the same predetermined elution time for the first sample in (d).

5. The method of any of claims 1 to 4, wherein the protein in the second eluate is enzymatically digested in (1), optionally wherein enzymatic digestion of the protein comprises contacting, via the FIA, the second eluate with a proteolytic enzyme selected from: trypsin, chymotrypsin, pepsin, thermolysin, papain, pronase, endopeptidase Arg-C, peptidyl-Asp metallo endopeptidase (endopeptidase Asp-N), Glutamyl endopeptidase (Glu-C endopeptidase), and Lysyl endopeptidase (Lys-C endopeptidase).

6. The method of claim 5, wherein (1) comprises:
(i) introducing, via the FIA, a digestion reagent to form a digestion mixture, wherein the second eluate is contacted with an amount of proteolytic enzyme determined by the at least one processor based on the concentration of the first eluate determined in (g);
(ii) mixing the digestion mixture; and
(iii) incubating the digestion mixture.

7. The method of claim 6, wherein the digestion reagent comprises a digestion buffer with a pH from 6.0 to 7.5, and, optionally, wherein (i) the digestion reagent comprises a reducing agent comprising Tris(2-carboxyethyl)phosphine (TCEP) and/or a proteolytic enzyme, optionally trypsin; and/or (ii) wherein incubating the digestion mixture comprises incubating at a temperature from 36°C to 85°C for 5 minutes to 30 minutes.

8. The method of claim 7, wherein the proteolytic enzyme comprises heat-stable trypsin, the method comprising:
(i) introducing, via the FIA, a first amount of digestion buffer, reducing agent and heat-stable trypsin to the second eluate and mixing to form a first digestion mixture;
(ii) incubating the first reaction mixture at a temperature of 70°C to 75°C for 10 minutes to 20 minutes to form a first incubated mixture;
(iii) introducing, via the FIA, a second amount of heat-stable trypsin, optionally in a second amount of digestion buffer, to the incubated mixture and mixing to form a second digestion mixture; and
(iv) incubating the second digestion mixture at a temperature of 70°C to 75°C for 10 minutes to 20 minutes to form a peptide mixture.

9. An automated method for monitoring post-translational modifications of a recombinantly produced protein which is an antibody, antibody fragment, or Fc fusion protein, the method to be carried out by a flow injection analyzer (FIA) comprising a syringe pump and a multi-port valve controlled by at least one processor, the method comprising:
(a) receiving a first sample that includes the protein in a holding reservoir of the FIA;
(b) introducing, by action of the multi-port valve, a first volume of a binding buffer into a protein A affinity column in the FIA;
(c) advancing, by action of a syringe pump, the first sample from the holding reservoir to the affinity column and allowing the protein in the first sample to bind to the affinity column;
(d) introducing, by action of the multi-port valve, a first volume of an elution buffer into the affinity column to elute the bound protein to form a first eluate, wherein the elution time of the protein in the first sample eluted from the affinity column in the first eluate is controlled by the at least one processor based on a predetermined elution time;
(e) advancing, by action of the syringe pump, the first eluate to a spectrophotometer in the FIA;
(f) determining, by the at least one processor and according to a measured UV absorbance, a concentration of protein in the first eluate according to a predetermined calibration curve;
(g) receiving a second sample that includes the protein in the holding reservoir;
(h) introducing, by action of the multi-port valve, a second volume of the binding buffer into the affinity column;
(i) advancing, by action of the syringe pump, the second sample from the holding reservoir to the affinity column and allowing the protein in the second sample to bind to the affinity column;
(j) introducing, by action of the multi-port valve, a second volume of elution buffer into the affinity column to elute the bound protein to form a second eluate, wherein the elution time of the protein in the second sample eluted from the affinity column in the second eluate is controlled by the at least one processor based on the same predetermined elution time for the first sample in (d);
(k) advancing, by action of the syringe pump, the second eluate to a digestion chamber in the FIA; and
(l) introducing, by action of the multi-port valve, a digestion reagent into the second eluate in the digestion chamber and digesting the protein to form a peptide mixture wherein an amount of proteolytic enzyme is determined by the at least one processor based on the concentration of the first eluate determined in (g); and
(m) advancing, by action of the syringe pump, the peptide mixture to a peptide mapping unit and detecting a post-translational modification of the protein.

10. The method of claim 9, wherein digesting the protein in (1) comprises:
(i) introducing, by action of the multi-port valve, a digestion buffer, a reducing agent, and a proteolytic enzyme into the second eluate in the digestion chamber and mixing to form a first digestion mixture;
(ii) incubating the first digestion mixture in the digestion chamber to form a first incubated mixture;
(iii) introducing, by action of the multi-port valve, a second volume of digestion buffer and proteolytic enzyme into the incubated mixture to form a second digestion mixture; and
(iv) incubating the second digestion mixture in the digestion chamber to form a peptide mixture;
wherein the proteolytic enzyme optionally comprises trypsin.

11. The method of claim 10, wherein the digestion buffer has a pH from 6.0 to 7.5; wherein the first digestion mixture in (ii) is incubated at a temperature from about 36°C to about 85°C for about 5 minutes to about 30 minutes; and wherein the second digestion mixture in (iv) is incubated at a temperature from about 36°C to about 85°C for about 5 minutes to about 30 minutes, optionally wherein the reducing agent comprises Tris(2-carboxyethyl)phosphine (TCEP), and optionally wherein the proteolytic enzyme comprises a heat stabile trypsin.

12. The method of claim 10, wherein the proteolytic enzyme comprises heat-stable trypsin, the method comprising:
(i) introducing, by action of the multi-port valve, a first amount of digestion buffer, reducing agent and heat-stable trypsin into the second eluate and mixing to form a first digestion mixture;
(ii) incubating the digestion mixture at a temperature of 70°C to 75°C for 10 minutes to 20 minutes to form a first incubated mixture;
(iii) introducing, by action of the multi-port valve, a second amount of digestion buffer and heat-stable trypsin to the incubated mixture and mixing to form a second digestion mixture; and
(iv) incubating the second digestion mixture at a temperature of 70°C to 75°C for 10 minutes to 20 minutes to form a peptide mixture.

13. The method of any one of claims 1-12, wherein the peptide mapping unit comprises a mass spectrometer (MS), liquid chromatography (LC) column, and a ultraviolet (UV) detector; wherein detecting post-translational modifications comprises a peptide mapping analysis performed using mass spectrometry (MS), liquid chromatography (LC), and ultraviolet (UV) detection, wherein liquid chromatography optionally comprises ultra-performance liquid chromatography (UPLC); and wherein the post-translational modification comprises glycosylation, optionally N-linked glycosylation, O-linked glycosylation or a combination thereof, oxidation, deamidation, isomerization, glycation, N-terminus leader sequence, N-terminal cyclization, C-terminus lysine retention, amide formation or a combination thereof.

14. An automated system for monitoring a post-translational modification of a recombinantly produced protein which is an antibody, antibody fragment, or Fc fusion protein, the system comprising:
(a) a holding reservoir configured to receive a first and a second sample that includes the protein;
(b) a first syringe pump configured to advance the first and second sample from the holding reservoir through a flow injection analyzer (FIA);
(c) a first multi-port valve in fluid communication with first syringe pump;
(d) a protein A affinity column in fluid communication with the first multi-port valve and configured to receive the first and second sample from the holding reservoir;
(e) a spectrophotometer configured to receive a first eluate from the affinity column, wherein the first eluate comprises protein from the first sample, wherein the spectrophotometer is configured to determine a UV absorbance, and calculate a protein concentration of the first sample based on a predetermined calibration curve;
(f) a second syringe pump configured to advance a second eluate from the affinity column to a digestion chamber, wherein the second eluate comprises protein from the second sample, and wherein the protein in the second eluate is digested based on the protein concentration of the first sample determined in (e); and
(g) a second injection multi-port valve in fluid communication with the second syringe pump.

15. A method of producing a recombinant therapeutic protein comprising antibody, antibody fragment, or Fc fusion protein, the method comprising:
(a) culturing a mammalian host cell in a bioreactor comprising a batch, fed-batch or perfusion bioreactor under conditions in which the recombinant protein is expressed;
(b) detecting a post-translational modification of the protein according to the method of any of claims 1 to 14 or 16;
(c) modifying one or more cell culture parameters based on the post-translational modification detected in (b) wherein optionally the one of more of the following cell culture parameters is pH; CO₂ level; amount of dissolved oxygen (dO₂); temperature; amount or type of nutrient; presence and types of glycan precursors, or a combination thereof; and
wherein the host cell is optionally a CHO, HEK 293, COS, NS0, SP2 or PER.C6 cell.

16. A recombinant protein produced by the method of claim 15 or a pharmaceutical composition comprising the recombinant protein produced by the method of claim 15 and a pharmaceutically acceptable carrier.
